(19) Europäisches Patentamt
European Patent Office
Office européen des brevets

(11) Numéro de publication: **0 462 264 B1**

# (12) FASCICULE DE BREVET EUROPEEN

(45) Date de publication de fascicule du brevet: **01.03.95**

(51) Int. Cl.[6]: **C07D 417/12**, A61K 31/425, C07D 417/14, C07D 413/12, A61K 31/44, A61K 31/41

(21) Numéro de dépôt: **91902715.1**

(22) Date de dépôt: **28.12.90**

(86) Numéro de dépôt internationale : **PCT/FR90/00970**

(87) Numéro de publication internationale : **WO 91/09857 (11.07.91 91/15)**

## (54) DERIVES HETEROCYCLIOUES, LEUR PROCEDE DE PREPARATION ET LEUR APPLICATION THERAPEUTIOUE COMME ANTAGONISTES DU PAF.

(30) Priorité: **29.12.89 FR 8917491**

(43) Date de publication de la demande: **27.12.91 Bulletin 91/52**

(45) Mention de la délivrance du brevet: **01.03.95 Bulletin 95/09**

(84) Etats contractants désignés: **AT BE CH DE DK ES FR GB GR IT LI LU NL SE**

(56) Documents cités:
**EP-A- 0 149 884**
**EP-A- 0 283 390**
**DE-C- 880 749**

(73) Titulaire: **SANOFI**
**32-34, rue Marbeuf**
**F-75008 Paris (FR)**

(72) Inventeur: **BERNAT, André**
**14, chemin de Maurens**
**F-31270 Cugnaux (FR)**
Inventeur: **HERBERT, Jean-Marc**
**4, rue du Cantou-Caout**
**F-31830 Plaisance-du-Touch (FR)**
Inventeur: **VALETTE, Gérard**
**8, rue de Montségur**
**F-31120 Lacroix (FR)**

(74) Mandataire: **Polus, Camille et al**
**c/o Cabinet Lavoix**
**2, Place d'Estienne d'Orves**
**F-75441 Paris Cedex 09 (FR)**

EP 0 462 264 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des dérivés hétérocycliques, leur procédé de préparation et leurs applications thérapeutiques.

On a décrit dans EP-A-0 283 390 des dérivés d'amino-2 thiazole de formule

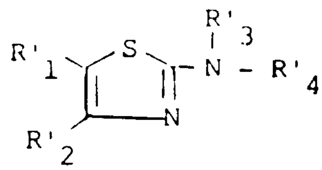

dans laquelle $R'_2$ représente, entre autres, un groupe aromatique, $R'_3$ représente H ou un groupe alkyle en $C_1$ à $C_4$ et $R'_4$ représente un groupe aminoalkyle dans lequel l'azote du groupe amino peut être notamment dans un hétérocyle tel que pyridine, pyrrolidine, pipéridine ou pipérazine; ces composés sont des agonistes des récepteurs muscariniques centraux et présentent une activité stimulante de la transmission cholinergique dans le système nerveux central.

Des amino-2 diphényl-4,5 oxazoles, disubstitués sur le groupe amino par hydroxyalkyl ou alkyl, ayant une activité antithrombotique ont été décrits dans FR-1 538009, tandis que ceux substitués sur le groupe amino par alkyl et hydroxyalkyl, ayant une activité antiinflammatoire, ont été décrits dans DE-2 518 882.

Parmi les dérivés d'amino-2 phényl-4 thiazoles-1,2,4, on peut citer les dérivés antimalariques décrits dans J. Het. Chem. 10, 611 (1973) dans lesquels la fonction amine est substituée par dialkylaminoalkyl et thiadiazolyl, ou des anesthésiques décrits dans CH-497 453 qui sont substitués sur la fonction amine par alkyl et aminoalkyl.

Parmi les dérivés d'amino-2 phényl-4 oxadiazole-1,2,4, on peut citer les anesthésiques locaux et vasodilatateurs décrits dans FR-2 148 430 qui sont disubstitués sur la fonction amine par dialkylaminoalkyl.

Les composés selon l'invention n'ont aucune de ces activités mais sont des antagonistes du PAF-acéther, (platelet activating factor); ils répondent à la formule

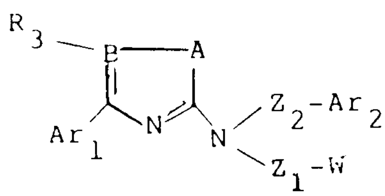

I

dans laquelle
A représente O ou S ;
B représente C ou N ;
$Z_1$ représente alkylène en $C_1$ à $C_4$ ou phénylène ;
$Z_2$ représente alkylène en $C_1$ à $C_4$ ;

W représente $NR_1R_2$, dans lequel $R_1$ représente H, alkyle en $C_1$ à $C_4$ et $R_2$ représente H, alkyle en $C_1$ à $C_4$, $CONQ_1Q_2$ ou $CSNQ_1Q_2$ dans lesquels $Q_1$ et $Q_2$ représentent indépendamment H ou alkyle en $C_1$ à $C_4$, $SO_2Q_3$ ou $COQ_3$ dans lesquels $Q_3$ représente alkyle en $C_1$ à $C_4$, $COOQ_4$ dans lequel $Q_4$ représente alkyle en $C_1$ a $C_4$ ou benzyle, ou $R_1$ et $R_2$ considérés ensemble forment avec N un hétérocyle saturé tel que morpholine, pyrrolidine, pipéridine, pipérazine ou ($C_1$-$C_3$)alkyl-4 pipérazine, ou W peut représenter le N-oxyde des amines $NR_1R_2$, ou encore W représente alkoxy ou thioalkoxy en $C_1$ à $C_4$, $CONQ_1Q_2$ ou $CSNQ_1Q_2$, pyridyle, imidazolyle ou $COOQ_5$ dans lequel $Q_5$ représente alkyle en $C_1$ à $C_5$ ;
$R_3$ n'est pas présent lorsque B est N, ou représente H, alkyle en $C_1$ à $C_8$ ou halogéno lorsque B est C ;
$Ar_1$ représente un phényle éventuellement substitué par un ou des groupes choisis parmi halogéno, alkyle, alkoxy ou thioalkoxy en $C_1$ à $C_4$, hydroxy, carboxy, $COOQ_6$ ou $COSQ_6$ ou $CSOQ_6$ dans lesquels $Q_6$ est un alkyle en $C_1$ à $C_4$, carboxamido, cyano, amino, acétamido, nitro, trifluorométhyle, ou $Ar_1$ représente un hétérocycle aromatique, thiényle, furyle, indolyle ou encore $Ar_1$ représente naphtyle benzyle ou cyclohexyle,
ou $Ar_1$ et $R_3$ considérés ensemble forment un groupe

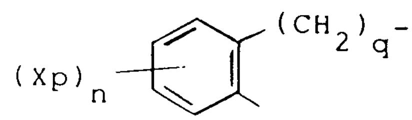

tel que le carbone du phényle soit lié en position 4 de l'hétérocycle et dans lequel q vaut 2 à 4, Xp identiques ou différents représentent H, alkyle en $C_1$ à $C_3$ ou halogèno et n représente 1 à 3;
$Ar_2$ représente un hétérocycle aromatique azoté, pyrimidinyle, quinolyle, isoquinolyle, indolyle, isoindolyle ou pyridyle, éventuellement substitués par alkyle ou alkoxy en $C_1$ à $C_3$ ou halogéno ;
les sels de ces composés avec des acides ou des bases sont également compris dans la définition des composés de l'invention; et lorsque A, B, $R_3$, $Ar_1$, $Ar_2$, $Z_1$ et $Z_2$ ont les significations suivantes :
A est S,
B est C,
$Z_1$ est défini comme ci-dessus,
$Z_2$ représente un groupe alkylène en $C_1$-$C_3$,
$R_3$ représente un atome d'hydrogène ou un groupe alkyle en $C_1$-$C_8$,
$Ar_1$ représente un groupe phényle portant éventuellement un ou plusieurs substituants choisis parmi les atomes d'halogène, les groupes alkyle ou alkoxy en $C_1$ à $C_4$, les groupes nitro, trifluorométhyle, ou hydroxy, ou $Ar_1$ et $R_3$ considérés ensemble forment le groupe

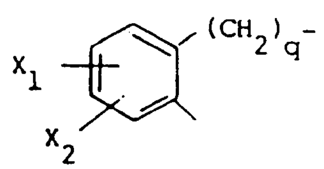

dans lequel q vaut 2 à 4, et $X_1$ et $X_2$ indépendamment l'un de l'autre représentent l'atome d'hydrogène, un halogène, ou un groupe méthyle, et
$Ar_2$ représente un groupe pyrimidinyle, quinolyle, isoquinolyle, indolyle ou pyridyle éventuellement substitués par un groupe alkyle ou alkoxy en $C_1$ à $C_3$ ou un atome d'halogène,
et W représente un groupe $NR_1R_2$ dans laquelle $R_1$ et $R_2$, indépendamment l'un de l'autre, représentent l'atome d'hydrogène, un groupe alkyle en $C_1$ à $C_4$ ou un phényle ou $R_1$ et $R_2$ considérés ensemble forment avec l'atome d'azote auxquels ils sont rattachés un hétérocyle saturé tel que morpholine, pyrrolidine, pipéridine, pipérazine, ou ($C_1$-$C_3$)alkyl-4 pipérazine, ou W représente un groupe pyridyle ou imidazolyle,

les sels d'addition de ces derniers composés avec des acides pharmaceutiquement acceptables sont inclus dans la définition des composés de l'invention.

Les groupes alkyle, alkylène, alkoxy, thioalkoxy sont droits ou ramifiés.

Les sels avec des acides ou des bases pharmaceutiquement acceptables sont préférés mais ceux qui peuvent permettre d'isoler les composés de formule I, notamment de les purifier, sont aussi objets de l'invention.

On préfère les composés dans lesquels B représente un atome de carbone et particulièrement les dérivés du thiazole dans lesquels $Ar_1$ représente un noyau phényle au moins ortho substitué ou ceux dans lesquels $Ar_1$ représente un phényle sans substituant en ortho et $R_3$ représente halogéno ou alkyle en $C_1$ à $C_3$ ; W est de préférence alkoxy ou thioalkoxy en $C_1$ à $C_2$ ou encore $NR_1R_2$ dans lequel $R_1$, $R_2$ représentent indépendamment H ou alkyle en $C_1$ à $C_4$ ou $NR_1R_2$ forment un hétérocyle saturé ; enfin, on préfère que $Z_1$ représente éthylène ou propylène.

Parmi ceux-ci, un groupe préféré de composés peut être représenté par la formule VIII :

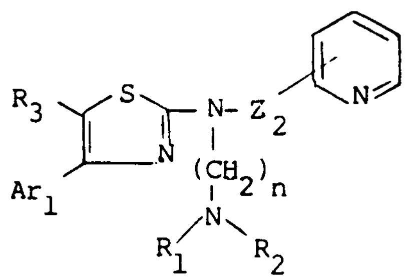

II

dans laquelle $R_1$ et $R_2$ représentent chacun un atome d'hydrogène ou alkyle en $C_1$ à $C_3$, ou forment ensemble avec l'atome d'azote auquel ils sont attachés pyrrolidinyle ou pipéridino, $Z_2$ représente $CH_2$ ou $CHCH_3$, $R_3$ représente H et $Ar_1$ représente un groupe phényle portant au moins un substituant en ortho ou $R_3$ représente Cl, Br ou alkyle en $C_1$ à $C_6$ et $Ar_1$ représente un phényle, éventuellement substitué, les substituants des groupes phényle étant choisis parmi les atomes d'halogène et les groupes alkyle ou alkoxy en $C_1$ à $C_4$, et n vaut 2 ou 3.

Comme on le verra plus loin des composés présentant un intérêt particulier, répondent à la formule

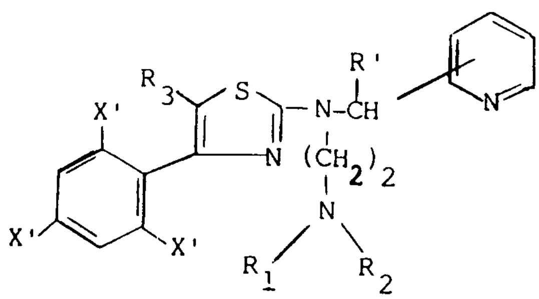

III

dans laquelle $R_3$ représente H ou halogéno tel que Br ou Cl, R' représente H ou $CH_3$, X' représente un alkyle en $C_1$ à $C_4$, et $R_1$ et $R_2$ représentent chacun séparément H ou alkyle en $C_1$ à $C_3$ ou ensemble avec l'atome d'azote auquel ils sont attachés pyrrolidinyle ou pipéridino et le groupe pyridyle étant substitué en position 2 ou 3.

Un autre groupe de composés préférés est constitué des composés pour lesquels $Ar_2$ représente un groupe pyridyle.

Parmi ces composés sont particulièrement préférés ceux pour lesquels $Ar_2$ représente un groupe pyridyl-2 ou -3, $Z_1$ représente un groupe alkylène en $C_2$ à $C_4$ et W représente le groupe $NR_1R_2$.

Parmi les composés préférés, on peut citer par exemple le N-[N',N'-diméthylamino-2 éthyl] N-[pyridyl-3 méthyl]amino-2 (triisopropyl-2,4,6 phényl)-4 thiazole, le N-[N',N'-diméthylamino-2 éthyl] N-[pyridyl-3 méthyl]amino-2 (triméthyl-2,4,6 phényl)-4 thiazole, les N-[N',N'-diméthylamino-2 éthyl] N-[pyridyl-3 méthyl]-amino-2 (dichloro-2,4 phényl)-4 méthyl-5 thiazole et oxazole et les N-[N',N'-diméthylamino-2 éthyl] N-[pyridyl-3 méthyl]amino-2 (triisopropyl-2,4,6 phényl)-4 chloro (ou bromo)-5 thiazole.

Les procédés de préparation des composés de formule I sont un autre objet de l'invention.

Selon un premier aspect, les composés sont préparés par réaction de l'amine secondaire de formule IV avec l'hétérocycle de formule V comportant un substituant X nucléofuge, tel qu'un halogène ou un sulfonate selon le schéma réactionnel (a)

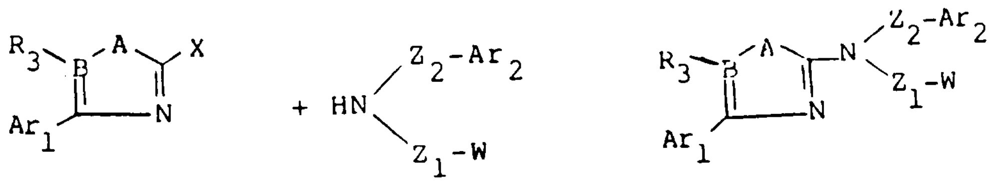

V IV I

dans lequel A, B, $R_3$, $Ar_1$, $Z_2$, $Ar_2$, $Z_1$, W ont les mêmes significations que pour la formule I, à l'exclusion de $R_3$ = halogène, ou $Ar_1$, $Ar_2$, W représentent des groupes correspondants dans lesquels les fonctions réactives ont été protégées de façon classique. Lorsque A représente O ou S et B représente C, on préfère que X soit Br, et la substitution est effectuée dans un solvant aprotique en général non polaire, tel qu'un hydrocarbure aliphatique ou aromatique, de préférence en présence d'une base pour neutraliser l'acide formé; lorsque A représente O ou S et B représente N, on préfère que X soit Cl, et la substitution est effectuée dans un solvant inerte tel qu'un alcool, un hydrocarbure aliphatique ou aromatique, une cétone ou un solvant chloré, comme le chlorure de méthylène.

On peut aussi préparer les composés de formule I à partir des composés de formule V en 2 étapes, en faisant réagir tout d'abord une amine primaire $Ar_2$-$Z_2$-$NH_2$ ou W-$Z_1$-$NH_2$ puis un halogénure ou un sulfonate respectivement de formule W-$Z_1$-Y ou $Ar_2$-$Z_2$-Ysur l'amine secondaire obtenue de préférence en présence d'une base. Lorsque A représente O et B représente N, dans le composé de formule V utilisé dans la première étape X peut représenter $CCl_3$.

Dans le cas où les composés de formule I comportent le thiazole comme hétérocycle, on peut aussi préparer directement l'aminothiazole convenablement substitué,

- soit par réaction d'une thiourée convenablement substituée avec une cétone alpha-halogénée
- soit par réaction de l'amine secondaire avec une alpha-thiocyanatocétone selon les schémas réactionnels (b)

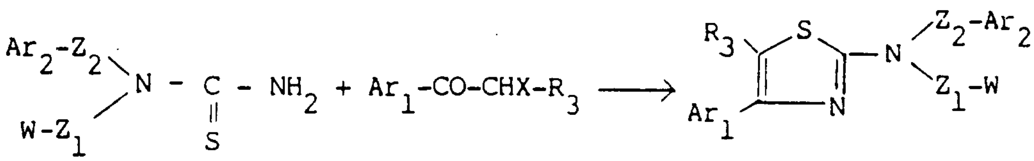

VI VII I

dans lequel W, $R_3$, $Z_1$, $Z_2$, $Ar_1$ et $Ar_2$ ont les mêmes significations que pour la formule I, à l'exclusion d'halogéno pour $R_3$, ou W, $Ar_1$, $Ar_2$ représentent des groupes correspondants dans lesquels les fonctions réactives ont été protégées de façon classique et X représente un atome d'halogène, notamment le chlore ou le brome et de préférence le brome,
et (c)

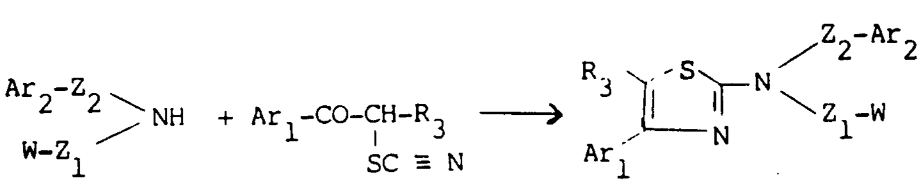

IV VIII I

dans lequel W, $R_3$, $Z_1$, $Z_2$, $Ar_1$ et $Ar_2$ ont les mêmes significations que pour la formule I, à l'exclusion d'halogéno pour $R_3$, ou W, $Ar_1$, $Ar_2$ représentent des groupes correspondants dans lesquels les fonctions réactives ont été protégées de façon classique.

Dans le cas (b), on peut éventuellement mettre en oeuvre le composé VII après avoir protégé la fonction carbonyle sous la forme d'un groupe acétal, qui sera éliminé lors de la condensation cyclisante ; les conditions réactionnelles sont celles généralement utilisées dans ce type de réaction, et on pourra se référer à la revue de G. Vernin, Heterocyclic compounds 34 /1/ 165-269 (1979) J.V. Metzger Ed. J. Wiley and Sons.

Plus particulièrement, la réaction est effectuée dans un solvant polaire, à une température comprise entre 40 °C et 100 °C. Parmi les solvants convenant on peut citer les alcools, tel que l'éthanol, le méthanol ou l'isopropanol, les acides aliphatiques, tel que l'acide acétique, les nitriles, tel que l'acétonitrile, les éthers tels que le dioxanne ou le tétrahydrofuranne. On ajoute de préférence un acide fort dans le milieu réactionnel pour accélérer la réaction, lorsque les thiourées de formule VI ne sont pas dégradées en milieu acide.

Dans le cas (c), de façon générale, une solution des composés IV et VIII dans un solvant inerte, tel qu'un hydrocarbure aliphatique ou aromatique, est maintenue quelques heures sous agitation à une température comprise entre la température ambiante, soit 20 °C environ, et la température de reflux du solvant choisi, et de préférence à une température comprise entre 60 °C et 110 °C.

Lorsque les composés de formules IV à VIII mis en oeuvre comportent des fonctions qui pourraient réagir dans les conditions opératoires utilisées pour la préparation des composés de formule I, celles-ci sont préalablement protégées; ainsi lorsque W représente $NR_1R_2$ et $R_1$ et/ou $R_2$ représente H, on peut bloquer la fonction amine sous forme de carbamate, notamment en fixant sur l'azote le groupement tert-butoxycarbonyle dont on sait que l'élimination s'effectue facilement par action d'un acide anhydre, tel que l'acide trifluoroacétique ou l'acide chlorhydrique ; lorsque $Ar_1$, $Ar_2$ ou W comportent une fonction acide, on prépare préalablement le composé de formule I comportant la fonction ester d'alkyle en $C_1$ à $C_4$ correspondante qui est hydrolysée, de façon usuelle, en milieu basique ou éventuellement acide ; lorsque $Ar_1$ ou $Ar_2$ comportent une fonction amine primaire, on peut préparer préalablement le composé de formule I comportant une fonction acétamide, que l'on hydrolyse en milieu acide ou basique.

Par ailleurs dans les cas où W représente $NR_1R_2$ avec $R_2$ représentant $CONQ_1Q_2$,$CSNQ_1Q_2$,$SO_2Q_3$,$COQ_3$, on peut préparer les composés de formule I à partir de ceux dans lesquels $R_2$ représente H.

Les composés de formule I dans lesquels $R_3$ est un atome d'halogène sont préparés par action de l'halogène, $Cl_2$, $Br_2$ ou ICl, sur le composé de formule I correspondant dans lequel $R_3$ représente H, en appliquant notamment la méthode décrite pour les thiazoles dans J. Am. Chem. Soc. 68 453-458 (1946).

Les produits de formule I sont isolés du milieu réactionnel et purifiés par les techniques classiques, en tenant compte de leurs propriétés physicochimiques et notamment de leur caractère basique. Certains de ces produits sont huileux, et on préfère alors les isoler sous forme d'un sel d'addition avec un acide minéral ou organique, sel qui est parfois hydraté ou solvaté; les sels sont préparés par l'action d'un acide sur le composé de formule I en solution, par exemple, dans un alcool ou un éther et sont isolés soit par précipitation, soit par évaporation du solvant; on peut salifier toutes les fonctions amines de la molécule ou seulement certaines, selon les conditions opératoires et l'acide mis en oeuvre.

Le N-oxyde de la fonction amine peut être préparé par action d'un agent oxydant convenable sur un composé de formule I notamment par action de la (phénylsulfonyl)-2 phényl-3 oxaziridine selon le procédé décrit dans J. Org. Chem. 53, p. 5856 - (1988) -.

Dans ces conditions les atomes d'azote de l'hétérocyle et de $Ar_2$ ne sont pas oxydés.

La plupart des produits de formule IV à VIII ne sont pas commerciaux et certains sont nouveaux, mais ils peuvent être préparés par des procédés dont le principe est connu en soi et dont la mise en oeuvre a été décrite pour des produits analogues.

Ainsi les amines secondaires de formule IV seront préparées à partir des amines primaires $Ar_2$-$Z_2$-$NH_2$ ou W-$Z_1$-$NH_2$, dans lesquelles les fonctions sensibles seront éventuellement protégées :

- soit par une réaction de substitution nucléophile dans laquelle on fait réagir sur l'amine primaire, selon le cas, un composé de formule $Ar_2$-$Z_2$-Y ou de formule W-$Z_1$-Y, dans lesquelles Y représente un atome d'halogène ou un groupement sulfonate $ZSO_3$- dans lequel Z représente un alkyle en $C_1$ à $C_4$ ou un phényle, éventuellement substitué.
- soit par la condensation de l'amine primaire avec une cétone ou un aldéhyde, en milieu déshydratant, suivie de la réduction de l'imine formée, de façon classique par un hydrure métallique ou par l'hydrogène en présence d'un catalyseur, comme Pd, en accord avec le schéma réactionnel suivant (d) :

$$R_4NH_2 + R_5COR_6 \longrightarrow R_4\text{-}N = CR_5R_6 \longrightarrow R_4\text{-}NH\text{-}CHR_5R_6$$

IV bis

dans lequel $R_4$ représente $-Z_2Ar_2$, auquel cas $-CHR_5R_6$ représente $-Z_1W$ ou $R_4$ représente $-Z_1W$ auquel cas $-CHR_5R_6$ représente $-Z_2Ar_2$, selon un procédé décrit notamment dans Methoden Org. Chemie IV /1d/ 355-363, (1981).

Les cétones de formule VII peuvent être préparées par halogénation des cétones de formule $Ar_1COCH_2R_3$, qui sont en général connues, et souvent commercialisées; dans le cas contraire, elles peuvent notament être preparées, par réaction de Friedel Crafts entre $Ar_1H$ et $R_3CH_2COCl$, en présence d'un acide de Lewis.

On prépare les cétones alpha-bromées à partir de $Ar_1COCH_2R_3$, notamment par action du brome dans un solvant tel que l'acide acétique, le tétrachlorure de carbone ou un éther tel que l'éther éthylique, par action du bromure cuivrique en appliquant la méthode décrite dans J. Org. Chem. 29 p. 3459-3461 (1964), par action de tribromures d'ammonium quaternaires, comme décrit dans Bull. Chem. Soc. Japan 60 1159-1160 et 2667-2668 (1987); les cétones alpha-chlorées peuvent être préparées par action des dichloroiodates d'ammonium quaternaire, comme décrit dans Synthesis p. 545-546 (1988).

Dans certains cas, il est avantageux de préparer les cétones alpha-bromées par une réaction de Friedel et Crafts entre le dérivé aromatique de formule $Ar_1H$ et le chlorure d'acide $R_3$-CHBr-COCl, en appliquant la méthode décrite dans Methoden der Org. Chemie VII /2a/ p. 110-132 (1977).

Enfin lorsque $R_3$ représente H, on peut partir du chlorure d'acide $Ar_1COCl$ sur lequel on fait réagir le diazométhane, réaction suivie de l'hydrolyse de la diazocétone obtenue par un hydracide, méthode décrite notamment dans Org. Synth. Coll. vol. 3 p. 119-120.

Les thiourées de formule VI peuvent être preparées par l'intermédiaire du composé de formule IX

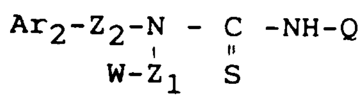

résultant de la réaction entre une amine secondaire de formule IV et un isothiocyanate de formule Q-N = C = S, dans lesquelles Q représente un groupe acyle éliminable en milieu acide, notamment acétyle, benzoyle ou pivaloyle et de préférence pivaloyle; le dérivé Q - N = C = S est lui-même préparé par action du chlorure d'acide carboxylique sur un thiocyanate métallique dans un solvant anhydre comme l'acétone ou la méthyléthylcétone.

On obtient le composé de formule VI par action d'un acide fort sur les composés de formule IX, par exemple par action d'une solution aqueuse de HCl à une température comprise entre 10 °C et 100 °C, et en particulier de HCl 12N.

Les thiocyanatocétones de formule VIII peuvent être preparées par action d'un thiocyanate métallique sur la cétone alpha-halogénée correspondante de formule VII dans un solvant anhydre en appliquant le procédé de Tcherniac, décrit notamment dans Heterocyclic compounds 34 (1) p. 271-273 (1979).

Les halogéno-2 thiazoles de formule V dans laquelle A est S et B est C, peuvent être préparés par action, en milieu anhydre, d'un acide halogénhydrique sur une solution de thiocyanatocétone de formule VIII, selon le procédé décrit dans Heterocyclic Compounds 34(1) p. 273-276 (1979).

Les halogéno-2 oxazoles de formule V, dans laquelle A est O et B est C, peuvent être préparés à partir des oxazoline-one-2, notamment par action du chlorure de phosphoryle, en présence d'une amine tertiaire, selon le schéma réactionnel (e)

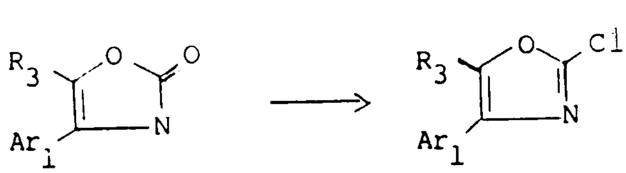

V

comme décrit dans Chem. Ber. 92 1928 (1959).

Pour les procédés de préparation des oxazolinones, on peut se référer à la revue de Y. S. Rao et R. Filler dans Heterocyclic Compounds 45 p. 660-665 (1986), I.J. Turchi, Ed. J. Wiley and Sons. On peut, en

particulier les préparer par cyclodéshydratation des carbamates de formule X selon le schéma réactionnel (f)

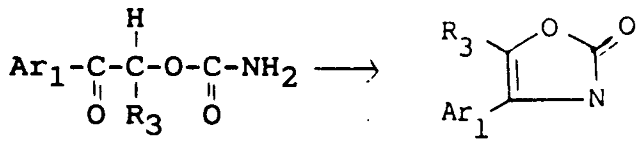

X

les carbamates étant eux-mêmes obtenus à partir des cétones alphahydroxylées $Ar_1$ CO CHOH-$R_3$.

Les chloro-5 thiadiazoles-1,2,4 peuvent être préparés par action du perchlorométhylmercaptan sur l'amidine $Ar_1C(NH)NH_2$, selon un procédé connu décrit notamment dans Chem. Ber. 90 182-187 (1957).

Les chloro-5 oxadiazoles-1,2,4 peuvent être préparés par action d'un agent de chloration sur les oxadiazoline-1,2,4 one-5, notamment par action de l'oxychlorure de phosphore en présence d'une amine, comme décrit dans Yakugaku Zasshi 84 1061-1064 (1964) (Chem. Abs. 62 5270d).

Enfin les trichlorométhyl-5 oxadiazoles-1,2,4 peuvent être préparés par action de l'anhydride trichloroacétique sur l'amidoxime $Ar_1C(NOH)NH_2$, comme décrit dans Helv. chem. Acta 46 1067-1073 (1963).

Des exemples de préparation de composés intermédiaires et de composés de formule I, ainsi que leurs caractéristiques physico-chimiques sont donnés plus loin.

Le spécialiste sera à même de choisir le procédé le plus adapté à la préparation d'un composé donné de formule I en tenant compte des matières premières disponibles, de la réactivité des intermédiaires et de leur stabilité.

Les composés de formule I et leurs sels sont des antagonistes du PAF-acéther. Ce phospholipide est un médiateur biologique dont la structure chimique a été déterminée en 1979, comme étant celle de la 1-0-hexadécyl 2-O-acétyl-sn-glycéro-3-phosphorylcholine, mais dont la libération par les basophiles au cours des réactions anaphylactiques avait été mise en évidence dès 1972. Ce médiateur a de nombreuses activités physiologiques parmi lesquelles on peut citer un effet potentialisateur de l'agrégation des plaquettes, un effet constricteur de la musculature lisse et des bronches, et un effet proinflammatoire ainsi qu'une activité hypotensive.

On peut se référer à l'article publié dans ISI Atlas of Science : Pharmacology 1(3) p. 187-198 (1987) qui fait le point sur les activités physiologiques du PAF et sur les applications thérapeutiques des antagonistes de ce phospholipide, incriminé dans de nombreuses pathologies, au moins comme facteur aggravant.

On connaît différentes méthodes pour mettre en évidence une activité antagoniste du PAF.

Ainsi in vitro, on peut étudier l'inhibition de l'agrégation de plaquettes de lapin induite par le PAF-acéther selon la méthode décrite dans Thrombosis Research 41 211-226 (1986). Dans cet essai, la plupart des composés de formule I, ou leurs sels, ont des $CI_{50}$ (concentration inhibant 50% de l'agrégation induite par le PAF introduit dans le milieu à la concentration de $4x10^{-10}$M) inférieures à $10^{-6}$M et pour de nombreux produits de l'ordre de $10^{-9}$M, alors que dans les mêmes conditions le composé appelé WEB 2086, antagoniste connu décrit dans J. Pharmacol. Exper. Therap. 241, 974-981 (1987), a une $CI_{50}$ de $5x10^{-8}$M. Parmi les composés les plus actifs, dont la $CI_{50}$ est de l'ordre de $10^{-9}$M, et qui présentent une durée d'action plus longue que le WEB 2086, on peut citer ceux de formule II explicitée précédemment.

Dans les mêmes conditions expérimentales, les composés de formule I, à la concentration de $10^{-4}$M sont inactifs sur l'agrégation induite par l'acide arachidonique à la concentration de $10^{-4}$M ou par l'adénosine -5' diphosphate, à la concentration de $10^{-5}$M.

Différentes méthodes sont aussi utilisées pour mettre en évidence chez l'animal l'inhibition des effets du PAF par des antagonistes, dont celle du bronchospasme induit chez le cobaye, qui est décrite dans Thromb. Haemostas. 56(1) 40-44 (1986) et celle de la protection contre le choc systémique léthal chez la souris qui est décrite dans J. Pharmacol. Exp. Ther. 247 (2) 617-23 (1988).

A titre d'exemple, on peut indiquer que les composés de formule III explicités ci-dessus, inhibent totalement le bronchospasme résultant de l'injection par voie intra-veineuse (IV) de 100 ng/kg de PAF, lorsqu'ils sont administrés 1 heure avant l'injection, soit IV à la dose de 1 mg/kg soit par voie orale à la dose de 3 mg/kg; ces mêmes composés diminuent de 50% la mortalité des souris, s'ils sont administrés 1 heure avant l'injection IV de 100 µg/kg de PAF, à des doses de l'ordre de 0,5 mg/kg par voie IV et de 10 mg/kg par voie orale. Parmi ceux-ci, le N-[N',N'-diméthylamino-2 éthyl] N-[pyridyl-3 méthyl]amino-2

(triisopropyl-2,4,6 phényl)-4 thiazole inhibe totalement le bronchospasme induit chez le cobaye à la dose de 1 mg/kg IV ou de 3 mg/kg per os pendant plus de 96 heures.

Par ailleurs, à la dose de 5 mg/kg per os, les composés

- N-[N',N'-diméthylamino-2 éthyl] N-[pyridyl-3 méthyl]amino-2 (triméthyl-2,4,6 phényl)-4 thiazole
- N-[N',N'-diméthylamino-2 éthyl] N-[pyridyl-3 méthyl]amino-2 (triisopropyl-2,4,6 phényl)-4 chloro-5 (ou bromo-5)thiazole.
- N-[N',N'-diméthylamino-2 éthyl] N-[pyridyl-3 méthyl]amino-2 (dichloro-2,4 phényl)-4 méthyl-5 thiazole (ou oxazole)

protègent la souris du choc systémique léthal induit par le PAF à la dose de 100 $\mu$g/kg.

Ainsi, selon un autre aspect, l'invention concerne les compositions pharmaceutiques comprenant -comme principe actif- au moins un composé de formule I ou l'un de ses sels pharmaceutiquement acceptables, en association avec les excipients usuels pour une administration par voie orale, rectale, transmuqueuse ou parentérale. Les doses unitaires et journalières dépendront du composé, de la nature et là gravité de la maladie, du sujet, comme de la voie d'administration; en général par voie orale, la dose unitaire sera chez l'adulte de 5 mg à 500 mg, tandis que par voie intra-veineuse, elle sera de 0,05 mg à 10 mg, ces doses étant compatibles avec les doses auxquelles l'activité pharmacologique de ces composés se manifestent chez l'animal, sans effet toxique apparent.

Les compositions de l'invention seront utiles notamment pour le traitement de l'asthme, de certains états allergiques ou inflammatoires, de maladies cardiovasculaires dont l'athérosclérose, les thromboses, l'hypotension ou les arythmies, les ischémies cérébrales et cardiaques, et de diverses pathologies rénales dont les glomérulonéphrites, ou encore comme agent contraceptif.

On décrit, tout d'abord, dans ce qui suit la préparation de composés intermédiaires de formule IV à VIII. Les points de fusion mentionnés sont instantanés; les points d'ébullition ont été en général mesurés lors d'une distillation sous pression réduite.

Amines de formule IV

A) N,N-diisopropyl N'-(pyridyl-3 méthyl)éthanediamine.

($R_1$ = $R_2$ = $CH(CH_3)_2$; $Z_1$ = $(CH_2)_2$; $Z_2$ = $CH_2$; $Ar_2$ = pyridyl-3)

Sous atmosphère inerte, on ajoute 16,9 g de pyridyl-3 carboxaldéhyde à une solution de 25 g de N,N-diisopropyl éthanediamine dans 150 ml de toluène, en présence de tamis moléculaire 4Å.

Après 1 heure à température ambiante, on filtre le tamis et on évapore le solvant sous pression réduire vers 30 °C; on reprend l'huile résiduelle par 150 ml de méthanol anhydre et refroidit la solution entre 0 et 10 °C. On ajoute alors par portions 6g de borohydrure de sodium et on laisse revenir à température ambiante; après plusieurs heures d'agitation, on introduit dans le milieu 10 ml d'une solution aqueuse de HCl 1N, puis on ajoute une solution aqueuse de NaOH 10N jusqu'à pH 8, avant d'extraire le produit final par le chlorure de méthylène. La phase organique est séchée sur sulfate de sodium, filtrée et concentrée sous pression réduite vers 70 °C. L'huile obtenue est distillée sous vide. On obtient 30,1 g du composé attendu. Eb : 115 °C/5 Pa.

B) N,N-diméthyl N'-(pyridyl-3 méthyl) éthanediamine.

($R_1$ = $R_2$ = $CH_3$; $Z_1$ = $(CH_2)_2$; $Z_2$ = $CH_2$; $Ar_2$ = pyridyl-3)

Sous atmosphère inerte, on ajoute 4 g de N, N-diméthyléthanediamine à une solution refroidie de 4,7 g de pyridine-3 carboxaldéhyde dans 40 ml d'éthanol absolu en présence de tamis moléculaire 4Å.

Après 1 heure à température ambiante, on filtre le tamis et ajoute à la solution refroidie entre 0 °C et 10 °C, 1,82 g de borohydrure de sodium. après 12 heures d'agitation à température ambiante, on concentre le milieu sous pression réduite et verse 10 ml d'une solution aqueuse de HCl N sur le résidu; on ajoute ensuite une solution concentrée de KOH jusqu'à pH supérieur à 8 et extrait le milieu par l'éther éthylique; la phase organique est séchée et le solvant éliminé sous pression réduite. L'huile obtenue est distillée sous vide. On obtient 5,4 g du composé attendu. Eb = 84 °C/40 Pa.

C) N,N-diméthyl N'-[(pyridyl-3)-1 éthyl] éthanediamine

($R_1$ = $R_2$ = $CH_3$; $Z_1$ = $(CH_2)_2$; $Z_2$ = $CHCH_3$; $Ar_2$ = pyridyl-3)

On chauffe à reflux pendant environ 5 heures, dans un ballon surmonté d'un séparateur de Dean Stark, 30 g d'acétyl-3 pyridine, 28,4 g de N,N-diméthyl éthanediamine et 20 mg d'acide para-toluène sulfonique dans 300 ml de benzène anhydre. Après concentration sous pression réduite vers 50 °C, on reprend l'huile résiduelle par 300 ml de méthanol anhydre, et on ajoute à température inférieure à 10 °C, 10 g de borohydrure de sodium et on traite le milieu réactionnel dans les conditions décrites selon A. On obtient après distillation, 38 g du composé attendu. Eb = 96 °C/60 Pa.

D) N-(pipéridino éthyl) N-[(pyridyl-3)-1 éthyl]amine

$(NR_1R_2$ = 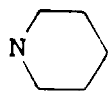 ;

$Z_1$ = $(CH_2)_2$; $Z_2$ = $CH\text{-}CH_3$; $Ar_2$ = pyridyl-3)

On chauffe à reflux durant 3 heures environ dans un ballon surmonté d'un séparateur de Dean Stark, 7,26 g d'acétyl-3 pyridine, 8 g de pipéridino éthylamine, 0,2 g d'acide para-toluène sulfonique dans 100 ml de toluène. On évapore sous vide le solvant vers 60 °C et on reprend l'huile dans 100 ml de méthanol anhydre; on ajoute ensuite vers 5 °C, 2,3 g de borohydrure de sodium par portions. Après une nuit d'agitation à température ambiante, on ajoute 10 ml d'acétone pour détruire l'excès de borohydrure et après 15 minutes, on ajoute 15 ml de solution aqueuse de NaOH 5N. Après concentration sous vide, on extrait le résidu par 3 fois 50 ml de chlorure de méthylène. Les phases organiques sont séchées sur sulfate de magnésium anhydre, filtrées et concentrées sous vide. On obtient après distillation 11,5 g du composé attendu. Eb = 115 °C/42 Pa.

E) N,N-[di(pyridyl-3)-1 éthyl]amine

W = pyridyl-3; $Z_1$ = $Z_2$ = $CHCH_3$; $Ar_2$ = pyridyl-3

Vers 5 °C, on ajoute par portions 8,8 g de cyanoborohydrure de sodium à une solution de 24,3g d'acétyl-3 pyridine et 157 g d'acétate d'ammonium dans 600 ml de méthanol et on laisse revenir vers 20 °C. Après environ 14 heures d'agitation à la température ambiante, on introduit dans le milieu 50 g d'acétyl-3 pyridine puis après quelques heures d'agitation, on introduit dans le milieu refroidit à 5 °C, 5g de cyanoborohydrure de sodium. On laisse revenir ensuite à température ambiante et après environ 14 heures d'agitation, on concentre à siccité vers 50 °C. Le résidu est alors acidifié sous hotte jusqu'à pH proche de 2 par addition d'une solution aqueuse d'HCl 12N, filtré et le filtrat est amené à pH 8 environ par addition d'une solution aqueuse de NaOH 10N. La phase aqueuse est extraite par l'acétate d'éthyle et après séchage de la phase organique et élimination du solvant, l'huile résiduelle est distillée sous vide, vers 140 °C à une pression de 21 Pa. On obtient ainsi 32 g d'amine. Le chlorhydrate préparé par action de HCl sur l'amine en solution dans 400 ml d'isopropanol fond à plus de 250 °C. On peut séparer les 2 diastéréoisomères par précipitation fractionnée dans le méthanol aqueux.

F) N,N-diméthyl N'-[(pyridyl-3)-2 éthyl]éthanediamine

($R_1$ = $R_2$ = $CH_3$; $Z_1$ = $(CH_2)_2$; $Z_2$ = $(CH_2)_2$; $Ar_2$ = pyridyl-3)

On chauffe durant environ 30 heures vers 60 °C, 11 g de' chlorhydrate de chloro-2 éthyl pyridine-3, 16,3 g de N,N-diméthyléthane diamine, 18,5 g de bicarbonate de sodium et 13 g d'iodure de potassium dans 150 ml d'éthanol. On évapore le solvant et reprend le résidu avec 100 ml d'eau, avant d'ajouter une solution aqueuse de NaOH 10N jusqu'à pH8; la phase aqueuse est alors extraite par 3 fois 80 ml d'acétate d'éthyle. Les phases organiques réunies sont séchées sur sulfate de magnésium, filtrées et concentrées sous pression réduite. L'huile résiduelle est distillée pour donner 6,7 g du produit attendu. Eb = 100-107 °C/10 Pa.

G) N-(tertiobutyloxycarbonyl) N-méthyl N'-(pyridyl-3 méthyl) éthanediamine

($R_1$ = $CH_3$; $R_2$ = $t\text{-}C_4H_9OCO$; $Z_1$ = $(CH_2)_2$; $Z_2$ = $CH_2$; $Ar_2$ = pyridyl-3)

Sous agitation, à un mélange de 6 g de N-méthyl N'-(pyridyl-3 methyl) éthanediamine, 95 ml de dioxanne, 20 ml d'eau et 1,74 g d'oxyde de magnésium, on ajoute très lentement, vers 3 °C, 5,9 g de dicarbonate de ditertiobutyle dans 60 ml de dioxanne. Après 15 minutes, on introduit dans le milieu 15 ml de soude environ 2N; le précipité est alors séparé et le filtrat est concentré sous pression réduite: le résidu est dissous dans environ 200 ml d'acétate d'éthyle puis séché sur carbonate de potassium et le solvant éliminé sous pression réduite après filtration des solides. L'huile résiduelle est purifiée par chromatographie sur silice en éluant avec de l'éther éthylique puis par un mélange de chlorure de méthylène/méthanol (95/5 v/v). Le N,N' dicarbamate est élué en tête, puis le produit recherché (poids 3,9 g) avant le carbamate formé sur l'azote portant le groupe methylpyridyle.

H) N-(pyridyl-3 méthyl) N'-(tertiobutyloxy-carbonyl) éthanediamine

($R_1$ = H; $R_2$ = $t\text{-}C_4H_9OCO$; $Z_1$ = $(CH_2)_2$; $Z_2$ = $CH_2$; $Ar_2$ = pyridyl-3)

On introduit 2,92 g de pyridyl-3 carboxaldéhyde dans une solution, vers 5 °C, de 4,82 g de N-(tertiobutyloxycarbonyl)éthanediamine dans 50 ml de toluène en présence d'un agent déshydratant (tamis moléculaire 4 Å).

La diamine peut être préparés selon la méthode décrite dans J. Med. Chem. 31 898 (1988). Après 4 heures à température ambiante, le tamis est séparé et le solvant est évaporé sous pression réduite. L'huile

résiduelle est dissoute dans 30 ml de méthanol anhydre, et on introduit dans la solution entre 0 °C et 5 °C, 0,68 g de borohydrure de sodium. Après 2 heures d'agitation à température ambiante, on introduit dans le milieu 10 ml d'acétone puis on évapore les solvants sous pression réduite. Le résidu est dissous dans 50 ml de chlorure de méthylène saturé d'eau avec 0,5 g de KOH; la phase organique est ensuite séchée, le solvant est éliminé sous pression réduite et le résidu est chromatograpié sur silice; l'élution est effectuée avec un mélange de chlorure de méthylène et méthanol (90/10- v/v).

On isole ainsi 5,1 g d'amine sous forme d'huile.

Les amines du Tableau I ont été préparées en appliquant l'un des modes opératoires ci-dessus.

TABLEAU 1

Amines IV bis : $R_4NHCHR_5R_6$

| $R_4$ | $R_5$ | $R_6$ | Ebullition °C/ pression Pa |
|---|---|---|---|
| N,N-diméthylamino-2 éthyl | pyridyl-2 | H | 99-101/70 |
| N,N-diméthylamino-2 éthyl | pyridyl-4 | H | 110/70 |
| N,N-diméthylamino-2 éthyl | pyridyl-4 | $CH_3$ | 80/40 |
| N,N-diméthylamino-4 butyl | pyridyl-3 | H | 137/50 |
| N,N-diméthylamino-1 propyl-2 | pyridyl-3 | H | 113/40 |
| N,N-diméthylamino-3 propyl | pyridyl-3 | H | 103-108/10 |
| N,N-diméthylamino-3 propyl | pyridyl-3 | $CH_3$ | 107-110/20 |
| morpholino-3 propyl | pyridyl-3 | H | 140/2 |
| (imidazolyl-1)-3 propyl | pyridyl-3 | H | 175/5 |
| (imidazolyl-1)-3 propyl | pyridyl-3 | $CH_3$ | 169/10 |
| N,N-diéthylamino-2 éthyl | pyridyl-3 | H | 104/10 |
| N,N-diéthylamino-2 éthyl | pyridly-3 | $CH_3$ | 103/6 |
| N,N-diisopropylamino-2 éthyl | pyridyl-3 | $CH_3$ | 116/7 |
| N,N-dibutylamino-2 éthyl | pyridyl-3 | $CH_3$ | 143/2 |
| (pyrrolidinyl-1)-2 éthyl | pyridyl-3 | H | 115-117/80 |
| (pyrrolidinyl-1)-2 éthyl | pyridyl-3 | $CH_3$ | 127/70 |
| morpholino-2 éthyl | pyridyl-3 | H | 123/5 |
| morpholino-2 éthyl | pyridyl-3 | $CH_3$ | 130/4 |
| pipéridino-2 éthyl | pyridyl-3 | H | 114/52 |
| méthyl-4 piperazinyl-2 éthyl | pyridyl-3 | H | 170-175/3 |
| N-méthyl N-phényl amino-2 éthyl | pyridyl-3 | H | 135/4 |
| (éthyl-1)pyrrolidinyl-2 éthyl | pyridyl-3 | $CH_3$ | 115/70 |
| pyridyl-2 méthyl | pyridyl-3 | H | 135/4 |
| pyridyl-3 méthyl | pyridyl-3 | H | 142/5 |
| N,N-diméthylamino-4 phényl | pyridyl-3 | H | 160-166/40 |
| N,N-diméthylamino-2 éthyl | quinolyl-3 | H | 165-170/40 |
| N,N-diméthylamino-2 éthyl | (N-méthyl)-indolyl-3 | H | 140/5 |
| N,N-diméthylamino-2 éthyl | (chloro-2) pyridyl-3 | H | 126/120 |
| méthoxy-2 éthyl | pyridyl-3 | H | 78/94 |
| éthylthio-2 éthyl | pyridyl-3 | H | 70/13 |
| N,N-diméthylcarbamoyméthyl | pyridyl-3 | H | 142/4 |

Thiourées de formule VI

I) N-(N',N'-diméthylamino-2 éthyl) N-(pyridyl-3 méthyl) thiourée

($R_1$ = $R_2$ = $CH_3$; $Z_1$ = $(CH_2)_2$; $Z_2$ = $CH_2$; $Ar_2$ = pyridyl-3)

i) - pivaloyl isothiocyanate : on ajoute lentement 18,7 g de chlorure de pivaloyle à une suspension, refroidie vers 4 °C, de 15,1 g de thiocyanate de potassium dans 150 ml d'acétone anhydre. On laisse sous agitation environ 3 heures à cette même température.

ii) - on ajoute lentement dans le milieu précédent, en maintenant une température inférieure à 10 °C, 20 g de N-(diméthylamino-2 éthyl) N-(pyridyl-3 méthyl) amine. Après une heure à température ambiante, on évapore le milieu à sec sous pression réduite vers 60 °C et on reprend le résidu dans le chlorure de méthylène, avant de laver avec une solution diluée d'ammoniaque; on évapore le solvant sous pression réduite et on reprend l'huile obtenue par environ 100 ml d'acide chlorhydrique concentré qu'on porte vers 80 °C durant 1 heure. Après refroidissement, on porte à pH 8 par addition d'une solution d'ammoniaque glacée, puis on extrait 3 fois par le chlorure de méthylène; on sèche les phases organiques sur sulfate de magnésium, on élimine les solides et on concentre sous pression réduite vers 60 °C. L'huile obtenue (41 g) peut être engagée telle quelle dans l'étape suivante ou purifiée par chromatographie sous moyenne pression sur silice (éluant : chlorure de méthylène/méthanol (8/2 v/v). Le produit pur cristallise dans l'acétate d'éthyle. F = 104 °C.

Les thiourées intermédiaires de synthèse des exemples ont été préparées en appliquant cette méthode; les points de fusion de certaines d'entre elles figurent dans le Tableau 2.

TABLEAU 2

Thiourées : 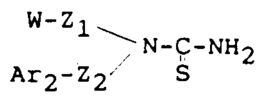

| $WZ_1$ | $Ar_2$-$Z_2$ | F °C |
|---|---|---|
| pyridyl-2 méthyl | pyridyl-3 méthyl | 143 |
| pyridyl-3 méthyl | pyridyl-3 méthyl | 140 |
| N,N-diméthylamino-2 éthyl | (pyridyl-3)-2 éthyl | 89 |
| N,N-diéthylamino-2 éthyl | pyridyl-3 méthyl | 78 |
| N,N-diisopropylamino-2 éthyl | pyridyl-3 méthyl | 86 |
| N,N-diisopropylamino-2 éthyl | (pyridyl-3)-1 éthyl | 122 |
| (pipéridinyl-1)-2 éthyl | pyridyl-3 méthyl | 107 |
| morpholino-2 éthyl | pyridyl-3 méthyl | 136-137 |
| morpholino-2 éthyl | (pyridyl-3)-1 éthyl | 145-146 |
| N,N-diméthylamino-3 propyl | pyridyl-3 méthyl | 70 |
| N,N-diméthylamino-3 propyl | (pyridyl-3)-1 éthyl | 90 |
| morpholino-3 propyl | pyridyl-3 méthyl | 146 |
| (imidazolyl-1)-3 propyl | pyridyl-3 méthyl | 136-138 |
| N,N-diméthylamino-4 phényl | pyridyl-3 méthyl | 180 |
| N,N-diméthylamino-4 butyl | pyridyl-3 méthyl | 82 |
| N,N-diméthylamino-2 éthyl | (chloro-2)pyridyl-3 méthyl | 162 |

Alpha-bromocétones de formule VII

J) (triméthyl-2,4,6)phényl (bromo-2) éthanone

(VII : $Ar_1$ = $(CH_3)_3$-2,4,6 $C_6H_2$ ; $R_3$ = H ; X = Br)

On introduit lentement 52 g de brome dans une solution, à 10 °C, de 50 g de (triméthyl-2,4,6 phényl) éthanone dans 100 ml d'acide acétique; Apres 1 heure d'agitation à cette température et 2 heures vers 20 °C le milieu réactionnel est versé dans un volume d'eau glacée et extrait par l'éther éthylique. La phase organique décantee est lavée par une solution aqueuse de $NaHCO_3$ à 5% puis à l'eau et après séchage sur $MgSO_4$ est concentrée sous pression réduite, vers 60 °C. On obtient 66g d'huile qui peut être purifiée par distillation ou cristallisation dans le pentane vers - 20 °C. F <50 °C.

K) (trifluorométhyl-2)phényl (bromo-2) éthanone

(VII : $Ar_1$ = $(CF_3)$-2$C_6H_4$; $R_3$ = H; X = Br)

On introduit, sous atmosphère inerte, 28,2 g de bromure cuivrique finement broyé dans une solution, au reflux, de 8,5 g de (trifluorométhyl-2)phényl éthanone dans 25 ml d'acétate d'éthyle et 25 ml de chloroforme; le milieu réactionnel est maintenu 3 heures à sa température de reflux, puis les solides sont

séparés et le filtrat est concentré puis distillé sous pression réduite. On obtient ainsi 10,7 g de cétone. Eb = 80 °C/3Pa.

L) (triisopropyl-2,4,6)phényl (bromo-2) éthanone

(VII : $Ar_1$ = $[(CH_3)_2CH]$-2,4,6 $C_6H_2$; $R_3$ = H; X = Br)

Sous atmosphère inerte, on introduit à 0 °C, dans une suspension de 16,5 g de chlorure d'aluminium anhydre dans 200 ml de dichloro-1,2 éthane, une solution de 25 g de triisopropyl-1,3,5 benzène dans 50 ml de dichloro-1,2 éthane.

Après 30 minutes d'agitation à cette température, on introduit lentement 20,7 g de chlorure de bromoacétyle dans le milieu réactionnel, puis on laisse revenir à température ambiante. après 5 heures d'agitation, on verse le milieu réactionnel dans 2 volumes d'un mélange d'eau et de glace (50/50); après 15 minutes on ajoute 1 volume de chlorure de méthylène et on décante la phase organique. Celle-ci est lavée avec une solution aqueuse de $NaHCO_3$ (6% p/V), puis à l'eau et séchée sur $MgSO_4$. Après élimination des solvants par distillation vers 70 °C, l'huile résiduelle est purifiée par distillation sous pression réduite. E = 116-124 °C/1Pa. On obtient 29,8 g de cétone qui fond à 56 °-58 °C.

M) (diméthyl-2,4 méthoxy-6)phényl (bromo-2)éthanone

(VII : $Ar_1$ = $(CH_3)_2$-2,4($OCH_3$)-6$C_6H_2$; $R_3$ = H; X = Br)

i) On introduit, à température inférieure à 10 °C, 2,9 g de chlorure d'acétyle dans un mélange de diméthyl-3,5 anisole et 4,9 g de chlorure d'aluminium anhydre dans 50 ml de dichloro-1,2 éthane. Après 3 à 4 heures d'agitation à température ambiante on verse le milieu réactionnel sur 2 volumes d'un mélange eau et glace. On ajoute alors un volume de chlorure de méthylène et on sépare la phase organique. Celle-ci est séchée puis concentrée sous pression réduite à une température de 60 °C environ. L'huile résiduelle est purifiée par distillation sous pression réduite. E = 81 °C/50Pa.

ii) Cette cétone est bromée par $CuBr_2$ en appliquant le mode opératoire K. F = 68 °C.

N) (ditertiobutyl-3,5 hydroxy-4)phènyl méthyl-3 (bromo-2) butanone-1.

Cette cétone est obtenue selon le procédé M à partir du chlorure d'isovaléryle; F = 110 °C; La cétone hydroxylée intermédiaire fond à 109 °C.

O) diméthyl-3,5(bromo-2 oxo-1 éthyl)-4 acétanilide

(VII : $Ar_1$ = $(CH_3)_2$-3,5 (NH-$COCH_3$)-4-$C_6H_2$; $R_3$ = H; X = Br).

Vers -5 °C, on introduit lentement dans une solution de 3,4 g de diméthyl-3,5-acétyl-4 acétanilide préparé selon la méthode décrite dans J. Org. chem. 18, 496-500 (1963) dans 40 ml de tétrahydrofuranne, 6,24 g de tribromure de phényltriméthylammonium en solution dans 40 ml de tétrahydrofuranne.

Après 30 minutes vers 5 °C, on ajoute à 20 °C, 3 ml d'une solution aqueuse d'hydrogénosulfite de sodium (0,5%-p/v) et 30 ml d'eau. La phase organique est séparée et la phase aqueuse extraite par 30 ml d'éther éthylique; Les solvants sont évaporés des phases organiques réunies; on isole ainsi 3 g du produit cherché fondant à 142 °C.

P) diméthyl-3,5 (bromo-2 oxo-1 éthyl)-4 benzonitrile

(VII : $Ar_1$ = $(CH_3)_2$-3,5 (CN)-4-$C_6H_2$: $R_3$ = H; X = Br).

On introduit lentement, vers -5 °C, 4,6 g de brome dans une solution de 4 g d'acétyl-4 diméthyl-3,5 benzonitrile préparé selon la méthode décrite dans J. chem. Soc. Perkin Trans. II, p. 943-949 (1988) dans 10 ml d'éther diéthylique et 5 ml de dioxanne. On laisse le mélange revenir à température ambiante, et après 1 heure d'agitation, on y introduit 15 ml d'une solution saturée de chlorure d'ammonium. La phase organique est séparée et le solvant en est éliminé sous pression réduite pour donner 4,8 g du produit cherché. F = 76 °C.

Les cétones alpha-bromes du tableau 3, préparées selon l'un des modes opératoires précédents, ont été isolées, les autres ont été utlisées brutes.

**TABLEAU 3**

$Ar_1-CO-CHBr-R_3$

| $Ar_1$ | $R_3$ | Procédé | Constante Physique |
|---|---|---|---|
| 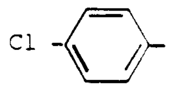 | $CH_3$ | I | F = 77°C |
| 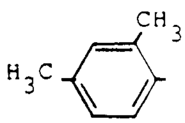 | $CH_3$ | I | Eb = 74°C/35 Pa |
| 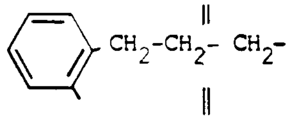 | | I | Eb = 120°C/45 Pa |
| 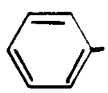 | $n-C_6H_{13}$ | J | Eb = 110°C/1Pa |
| 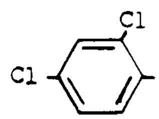 | H | J | F < 50°C |
| 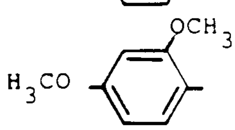 | H | J | F = 80°C |
| 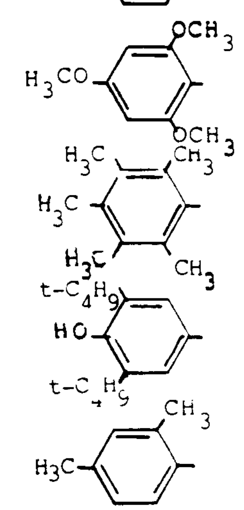 | H | J | F = 76°C |
| | H | J | F = 110°C |
| | H | J | F = 140°C |
| | H | K | Eb = 60°C/15 Pa |

TABLEAU 3 (suite)

| $Ar_1$ | $R_3$ | Procédé | Constante physique |
|---|---|---|---|
| 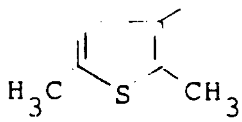 | H | J | F = 65°C |
| 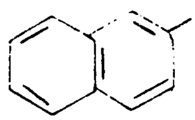 | H | O | F = 83°C |
| 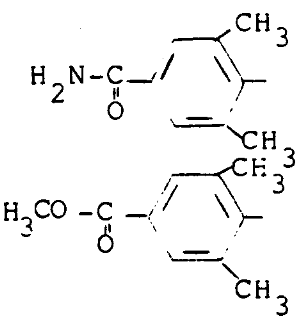 | H | P | F = 145°C |
|  | H | P | F = 117°C |
| 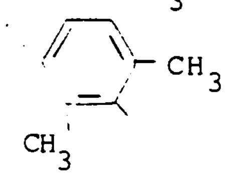 | H | P | F < 30°C |

Alpha-thiocyanato-cétones de formule VIII

Q) (triméthyl-2,4,6)phényl(thiocyanato-2) éthanone

On maintient vers 50 °C, pendant 3 heures, un mélange de 7 g de thiocyanate de potassium anhydre, 16 g de (triméthyl-2,4,6)phényl (bromo-2)éthanone et 180 ml d'acétonitrile. Le précipité formé est filtré vers 15 °C et le filtrat concentré sous pression réduite. On verse sur le résidu 250 ml d'éther éthylique, sépare le solide et élimine l'éther. L'huile résiduelle est dissoute dans l'éther isopropylique et la solution refroidie à 0 °C. Le précipité formé est séparé. On obtient ainsi 13,1 g du produit cherché qui fond à 73 °C.

En appliquant le même mode opératoire à la (triisopropyl-2,4,6)phényl (bromo-2) éthanone, on prépare la (triisoproyl-2,4,6) phényl thiocyanato-2 éthanone qui fond à 86 °C.

Halogéno-2 thiazoles de formule V

R) Bromo-2(triisopropyl-2,4,6 phényl)-4 thiazole

On refroidit à 0 °C une solution de 2 g de (triisopropyl-2,4,6) phényl thiocyanato-2 éthanone dans 80 ml d'acétonitrile et on sature à cette même température la solution avec de l'acide bromhydrique par barbotage. Le milieu est alors porté à 50 °C, température à laquelle il est maintenu pendant 2 heures en

atmosphère saturée d'acide bromhydrique, puis on refroidit jusqu'à 10°C et filtre le précipité apparu sous courant d'azote. On obtient ainsi 2,2 g du bromhydrate du composé attendu. F = 236°C.

En appliquant le même mode opératoire à la (triméthyl-2,4,6) phényl thiocyanato-2 éthanone, on obtient le bromhydrate du bromo-2 (triméthyl-2,4,6 phényl)-4 thiazole qui fond à 270°C.

En intoduisant dans le milieu réactionnel de l'acide chlorhydrique gazeux au lieu d'acide bromhydrique, on obtient le chlorhydrate du chloro-2 (triméthyl-2,4,6 phényl)-4 thiazole qui fond à 216°C.

Halogéno-2 oxazoles de formule V

S) chloro-2 (dichloro-2,4 phényl)-4 méthyl-5 oxazole

i- hydroxy-2 (dichloro-2,4 phényl)-1 propanone-1.

On introduit 26,1 g de phényl-iodosoacétate dans 150 ml d'une solution de 15 g de dichloro-2,4 propiophénone dans du méthanol anhydre, sous atmosphère inerte, puis vers 0°C, 12,4 g de KOH dans 60 ml de méthanol. Le milieu réactionnel est laissé 16 heures sous agitation à température ambiante puis le solvant est évaporé sous pression réduite. Le résidu est repris par 100 ml d'eau et 150 ml de chlorure de méthylène ; la phase organique est ensuite séparée, lavée à l'eau et le solvant évaporé. On verse sur le résidu 100 ml d'une solution aqueuse d'acide sulfurique à 5% (v/v) puis après 20 min., 100 ml de dichlorométhane sont ajoutés. Le phase organique est séparée et le solvant éliminé sous pression réduite. Le résidu est chromatographié sur silice en éluant à l'heptane, puis par un mélange heptane/acétate d'éthyle (99/1-v/v). On obtient ainsi 7 g du produit décrit.

ii- (dichloro-2,4 phényl)-4 méthyl-5 oxazolinone-2

On introduit sous atmosphère inerte dans une solution, vers -50°C, de 6 g de l'hydroxycétone obtenue selon i) et 8,2 ml de diméthylaniline dans 80 ml de toluène anhydre, une solution de 3,6 ml de trichlorométhyl-chloroformiate dans 20 ml de toluène anhydre. Après 1 heure d'agitation à cette température, on laisse le milieu revenir à température ambiante et l'y maintient pendant 5 heures. On fait alors barboter $NH_3$ gazeux dans le milieu durant 1 heure, puis on laisse sous agitation 2 heures avant d'éliminer le précipité. La phase organique est lavée à l'eau, séchée et concentrée sous pression réduite jusqu'à un volume de 20 ml environ. Le précipité formé est isolé, puis purifié par chromatographie sur silice en éluant avec un mélange d'éther isopropylique et d'heptane (8/2-v/v). On isole ainsi 3,1 g du produit cherché qui fond à 170°C.

iii- chloro-2 (dichloro-2,4 phényl)-4 méthyl-5 oxazole

3 g d'oxazolinone, obtenue en ii, sont dissous dans 15 ml d'oxychlorure de phosphore. A 0°C, on introduit dans cette solution 1,75 ml de triéthylamine, puis le mélange est maintenu 6 heures à sa température de reflux. Les produits volatils sont alors distillés sous pression réduite et le résidu est neutralisé par addition d'une solution aqueuse saturée de bicarbonate de sodium, vers 10°C. La phase aqueuse est extraite par 3 fois 80 ml d'acétate d'éthyle; les phases organiques réunies sont concentrées pour donner 2,8 g du produit attendu qui fond à 78°C.

On prépare selon les mêmes procédés la (méthyl-4 phényl)-4 méthyl-5 oxazolinone-2 qui fond à 169°C et le chloro-2 (méthyl-4 phényl)-4 méthyl-5 oxazole qui fond à moins de 50°C.

chloro-5 thiadiazoles de formule V

T) chloro-5 (triméthyl-2,4,6 phényl)-3 thiadiazole-1,2,4

i- triméthyl-2,4,6 benzamidine

On porte lentement jusqu'à 100°C, sous atmosphère inerte, 5 g de triméthyl-2,4,6 benzonitrile et 2,68 g d'amidure de sodium dans 80 ml de toluène, puis on laisse le mélange revenir à température ambiante et le laisse à cette température durant 16 heures environ. On introduit alors dans le milieu, refroidi vers 10°C, 10 ml d'éthanol puis 100 ml d'eau et 200 ml d'acétate d'éthyle. La phase organique est séparée, le solvant évaporé sous pression réduite et le résidu purifié par chromatographie sur silice en éluant au chlorure de méthylène puis avec une solution aqueuse de $NH_4OH$ à 25% (p/v) en mélange avec du méthanol (2/8-v/v). On obtient ainsi 2 g d'amidine fondant à 178°C.

ii- chloro-5 (trimèthyl-2,4,6 phényl)-3 thiadiazole-1,2,4

Dans une solution, vers -20°C, en atmosphère inerte de 2 g de triméthyl-2,4,6 benzamidine dans 16 ml de dichlorométhane, on introduit 2,2 g de chlorure de trichlorométhanesulfényle, puis lentement, vers -10°C, 2 g de NaOH en solution dans 3,2 ml d'eau. Après 1 heure vers -5°C, le précipité formé est isolé et la phase organique est séparée, et lavée avec 6 ml d'eau. Après séchage sur $K_2CO_3$, elle est concentrée sous pression réduite pour donner 2,8 g du produit cherché sous forme

d'huile, qui peut être utilisé sans autre purification.

chloro-5 oxadiazoles de formule V

U) chloro-5 (diméthyl-2,4 phényl)-3 oxadiazole-1,2,4

i- diméthyl-2,4 benzamidoxime

On maintient 16 heures au reflux 25 g de diméthyl-2,4 benzonitrile, 14,6 g de chlorhydrate d'hydroxylamine, 29 g de carbonate de potassium dans 150 ml d'éthanol et 35 ml d'eau. On ajoute alors 7,3 g de chlorhydrate d'hydroxylamine puis 14,5 g de carbonate de potassium et continue le reflux durant 5 heures.

Le milieu est ensuite concentré jusqu'à 70 ml par évaporation sous pression réduite, et on y ajoute 100 ml d'eau et 100 ml de chlorure de méthylène. La phase organique est séparée, et la phase aqueuse reextraite 2 fois par 100 ml de chlorure de méthylène. Le solvant est éliminé des 3 solutions organiques réunies, par distillation sous pression réduite puis le résidu est purifié par chromatographie sur colonne de silice en éluant avec des mélanges de cyclohexane et d'acétate d'éthyle (95/5-v/v puis 50/50-v/v). On isole ainsi 17 g de solide qui après recristallisation dans l'acétate d'éthyle fond à 150 ° C.

ii- (diméthyl-2,4 phényl)-3 oxadiazoline-1,2,4 one-5

Sous atmosphère inerte, on dissout 10,5 g de diméthyl-2,4 benzamidoxime dans 40 ml de toluène anhydre; on ajoute 6,5 ml de pyridine et 7,6 g de chloroformiate d'éthyle.

Le milieu est maintenu à sa température de reflux durant 5 heures, et le précipité formé à température ambiante est isolé puis redissous dans un mélange de 100 ml d'eau et 100 ml d'acétate d'éthyle; après agitation et décantation, la phase organique est séparée et concentrée sous pression réduite. On isole ainsi 7,6 g de produit attendu qui fond à 170 ° C.

iii- chloro-5 (diméthyl-2,4 phényl)-3 oxadiazole-1,2,4

On maintient 24 heures, vers 100 ° C, 2 g du produit obtenu selon ii avec 0,5 ml de pyridine, 0,5 ml de diméthylformamide et 15 ml d'oxychlorure de phosphore. Le milieu refroidi est versé sur 50 g de glace et la phase aqueuse formée est extraite par 3 fois avec 50 ml d'éther éthylique. Les phases organiques sont réunies, lavées à l'eau, séchées et concentrées sous pression réduite. Le résidu dissous dans un mélange heptane/acétate d'éthyle (90/10-v/v) est filtré sur silice. Après évaporation du solvant, on isole 1,3 g du composé attendu qui fond à 54 ° C.

On décrit dans ce qui suit des exemples de mise en oeuvre de l'invention; les spectres RMN ont été tracés à 250 MHz, sauf indication contraire; les déplacements chimiques sont exprimés en ppm par rapport au tétraméthylsilane (méthode de substitution); les points de fusion sont instanés.

EXEMPLE 1 : N-[(pyrrolidinyl-1)-2 éthyl] N-[pyridyl-3)-1 éthyl] amino-2 (triméthyl-2,4,6 phényl)-4 thiazole.
(formule I : A = S; B = C;$AR_1 = (CH_3)_3$-2,4,6—$C_6H_2$; $R_3$ = H; $Ar_2$ = pyridyl-3; $Z_1$ = $(CH_2)_2$; $Z_2$ = CH-$CH_3$; W = pyrrolidinyl-1).

On maintient environ 15 heures vers 70 ° C, sous atmosphère inerte, 2,4 g de N-[(pyrrolidinyl-1)-2 éthyl] N-[(pyridyl-3)-1 éthyl] thiourée, 2,04 g de (triméthyl-2,4,6 phényl)-1 bromo-2 éthanone et 0,3 ml de solution aqueuse de HCl environ 12N dans 60 ml d'éthanol. Le milieu est alors concentré par distillation sous pression réduite et on verse sur le résidu 50 ml d'une solution aqueuse de HCl N. Après lavage de la phase aqueuse par 2 fois 20 ml de chlorure de méthylène, on ajoute une solution aqueuse de NaOH 4N jusqu'à pH 8. On extrait ensuite par 3 fois 50 ml de chlorure de méthylène et après séchage, concentre les phases organiques.

L'huile résiduelle (3,7 g) est purifiée par chromatographie sur colonne de silice sous moyenne pression, en éluant avec un mélange d'acétate d'éthyle et de méthanol (50/50 puis 30/70 v/v). On obtient ainsi 3,05 g du produit cherché sous forme d'une huile.

RMN$^1$H(DMSOd6) amine : $\delta$ = 8,58(m,1H); 8,49(m,1H); 7,77(m,1H); 7,38(m,1H); 6,86(s,2H); 6,51(s,1H); 5,40-(q,1H); 3,42(m,2H); 2,65(m,1H); 2,37(m,5H); 2,23(s,3H); 2,03(s,6H); 1,63(m,7H).

Le chlorhydrate de ce composé est préparé en ajoutant à une solution de 2,85 g de l'huile dans 2 ml de méthanol anhydre, 11 ml d'une solution de HCl 2N dans $CH_3COOC_2H_5$. Le sel est isolé après élimination des solvants sous pression réduite. Après séchage à 50 ° C sous 0,1 Pa, on isole 3,57 g de trichlorhydrate monohydraté. F = 155 ° C.

EXEMPLE 2 : N-[N',N'-diméthylamino-2 éthyl] N-[pyridyl-3 méthyl] amino-2 (triisopropyl-2,4,6 phényl)-4 thiazole.
(formule I : A = S;B = C;$Ar_1$ = $[CH(CH_3)_2]_3$-2,4,6 $C_6H_2$; $R_3$ = H; $Ar_2$ = pyridyl-3; $Z_1$ = $(CH_2)_2$; $Z_2$ = $CH_2$; W = $N(CH_3)_2$).

On maintient environ 20 heures, sous atmosphère inerte, à la température de reflux, 9,8 g de (triisopropyl-2,4,6 phényl)-1 bromo-2 éthanone, 5 g de N-[pyridyl-3 méthyl] N-[N',N'-diméthylamino-2 éthyl] thiourée dans 100 ml d'éthanol anhydre. On distille ensuite les produits volatils sous pression réduite et on verse sur le résidu 100 ml d'une solution aqueuse de NaOH 2N; la phase aqueuse est extraite par 3 fois 100 ml de chlorure de méthylène et les phases organiques, après lavage à l'eau et séchage, sont concentrées sous pression réduite. L'huile résiduelle est dissoute dans 50 ml environ d'un mélange chlorure de méthylène/ méthanol (9/1-v/v) et filtrée sur silice. Le filtrat est amené à siccité et le résidu, dissout dans 30 ml d'acétone, est mélangé avec 50 ml d'une solution d'acide oxalique M dans l'acétone. Le précipité formé est isolé après 30 minutes. On obtient ainsi le dioxalate du produit final. F = 156-158 °C.

Les fonctions amines sont libérées par action de NaOH sur une solution aqueuse du dioxalate. Le produit cristallise dans l'éther de pétrole. F = 84 °C.
RMN$^1$H(DMSOd6) amine : $\delta$ = 8,55(s,1H); 8,47(m,1H); 7,7(m,1H); 7,33(m,1H); 7,00(s,2H); 6,51(s,1H); 4,74-(s,2H); 3,54(m,2H); 2,87(m,1H); 2,71(m,2H); 2,48(m,2H); 2,15(s,6H); 1,22 (d,6H); 1,06(d,12H).

On peut préparer le monofumarate par action de 1,2 équivalents d'acide fumarique sur une solution à 10% (p/v) de la base dans l'isopropanol. F = 195 °C.

Le monométhane sulfonate, qui cristallise avec 2 molécules d'eau, peut être préparé dans un mélange d'éther éthylique et d'isopropanol (10/1, v/v). F = 180 °C.

Le trimaléate préparé dans un mélange éther éthylique/acétone (2/7, v/v) fond à 115 °C.

Le trichlorhydrate, qui cristallise avec 3 molécules d'eau, peut être préparé dans l'éther éthylique. F = 140 °C.

Les composés de formule I dans laquelle A = S, B = C des exemples 3 à 63 qui figurent dans le tableau 4 ont été préparés en appliquant l'un des modes opératoires précédents. Les caractéristiques des spectres RMN de ces composés figurent dans le tableau 6 plus loin.

TABLEAU 4

| Ex. | $R_3$ | $Ar_1$ | $-Z_2-Ar_2$ | $-Z_1-W$ | amine ou sel de | F°C |
|---|---|---|---|---|---|---|
| 3 | H | 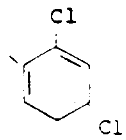 | 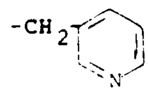 | $-(CH_2)_2-N(CH_3)_2$ | $2(COOH)_2$ | 182°C |
| 4 | H | 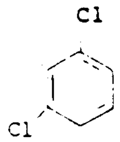 | 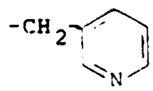 | $-(CH_2)_2-N(CH_3)_2$ | $2(COOH)_2$ | 165°C |
| 5 | H | 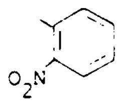 | 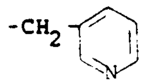 | $-(CH_2)_2-N(CH_3)_2$ | $2(COOH)_2$ | 182°C |
| 6 | H | 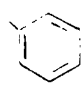 | 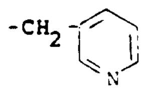 | $-(CH_2)_2-N(CH_3)_2$ | $1,5(COOH)_2$ $2H_2O$ | 150°C |
| 7 | H | 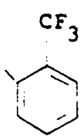 | 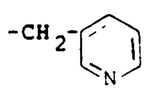 | $-(CH_2)_2-N(CH_3)_2$ | $2(COOH)_2$ | 131°C |
| 8 | $CH_3$ | 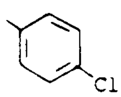 | 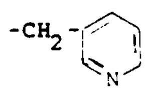 | $-(CH_2)_2-N(CH_3)_2$ | $3(COOH)_2$ | 176°C |
| 9 | $CH_3$ | 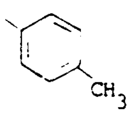 | 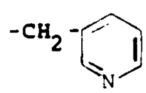 | $-(CH_2)_2-N(CH_3)_2$ | $2(COOH)_2$ $H_2O$ | 170°C |
| 10 | H | 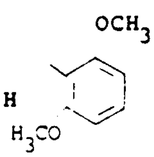 | 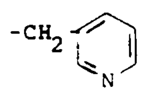 | $-(CH_2)_2-N(CH_3)_2$ | acide fumarique | 128°C |

TABLEAU 4 (suite 1)

| Ex. | $R_3$ | $Ar_1$ | $-Z_2-Ar_2$ | $-Z_1-W$ | amine ou sel de | F°C |
|---|---|---|---|---|---|---|
| 11 | H | 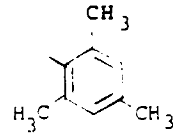 | 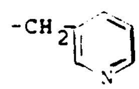 | $-(CH_2)_2-N(CH_3)_2$ | $(COOH)_2$ $4H_2O$ | 138°C |
| 12 | | 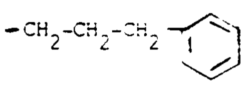 | 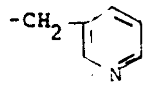 | $-(CH_2)_2-N(CH_3)_2$ | acide fumarique $0,5H_2O$ | 162°C |
| 13 | $CH_3$ | 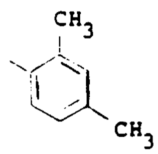 | 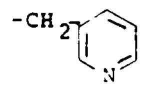 | $-(CH_2)_2-N(CH_3)_2$ | $1,5(COOH)_2$ | 154°C |
| 14 | H | 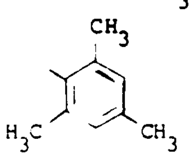 | 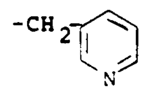 | $-(CH_2)_2-N(CH_3)_2$ | $3HCl,3H_2O$ | 215-220°C |
| 15 | H | 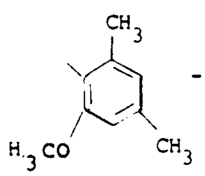 | 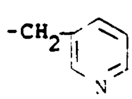 | $-(CH_2)_2-N(CH_3)_2$ | acide fumarique | 178°C |
| 16 | H | 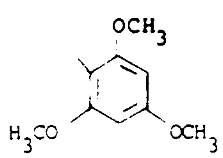 | 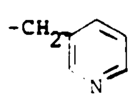 | $-(CH_2)_2-N(CH_3)_2$ | acide fumarique | 147°C |

TABLEAU 4 (suite 2)

| Ex. | $R_3$ | $Ar_1$ | $-Z_2-Ar_2$ | $-Z_1-W$ | amine ou sel de | F°C |
|---|---|---|---|---|---|---|
| 17 | H | 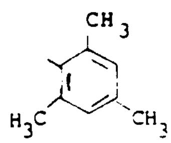 | 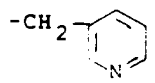 | $-(CH_2)_3-N(CH_3)_2$ | 3HCl, 2,5$H_2O$ | 130-135°C |
| 18 | H | 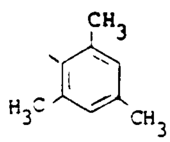 | 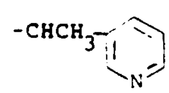 | $-(CH_2)_2-N(CH_3)_2$ | 3HCl, 2$H_2O$ | 190-195°C |
| 19 | H | 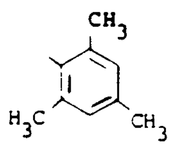 | 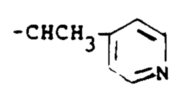 | $-(CH_2)_2-N(CH_3)_2$ | 3HCl, 3$H_2O$ | 188-190°C |
| 20 | H | 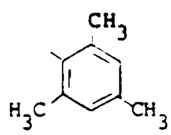 | 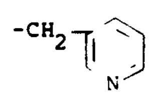 | $-CH(CH_3)-CH_2-N(CH_3)_2$ | 2(acide fumarique) | 168°C |
| 21 | H | 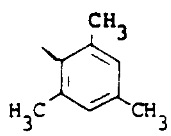 | 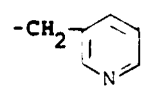 | 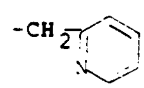 | 3HCl,$H_2O$ | 138°C |
| 22 | H | 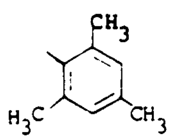 | 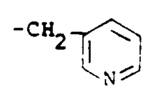 | 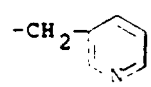 | 3HCl,2$H_2O$ | 210°C |
| 23 | H | 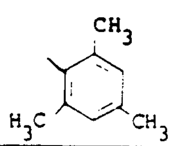 | 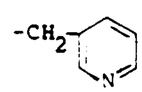 | 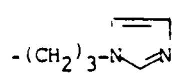 | 3HCl, 2,5$H_2O$ | 140-150°C |

TABLEAU 4 (suite 3)

| Ex. | $R_3$ | $Ar_1$ | $-Z_2-Ar_2$ | $-Z_1-W$ | amine ou sel de | F°C |
|---|---|---|---|---|---|---|
| 24 | H | 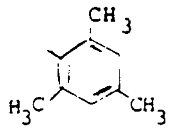 | $-CH_2-$ 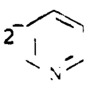 | $-(CH_2)_2-$ 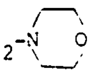 | 1,5(acide fumarique) | 151°C |
| 25 | H | 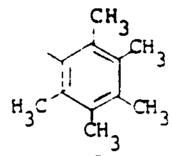 | $-CH_2-$ 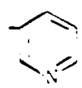 | $-(CH_2)_2-N(CH_3)_2$ | acide fumarique $H_2O$ | 166°C |
| 26 | H | 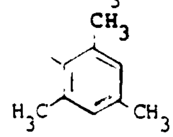 | $-CH_2-$ 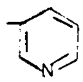 | $-(CH_2)_2-N(C_2H_5)_2$ | 3HCl, 2,5$H_2O$ | 170-180°C |
| 27 | H | 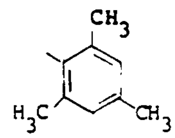 | $-CHCH_3-$ 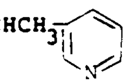 | $-(CH_2)_3-N(CH_3)_2$ | HCl, 3,5$H_2O$ | 135°C |
| 28 | H | 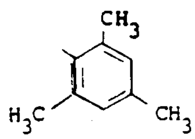 | $-CHCH_3-$ 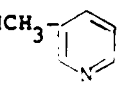 | $-(CH_2)_3-$ 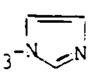 | 1,5(+)acide tartrique 5$H_2O$ | 90°C |
| 29 | H | 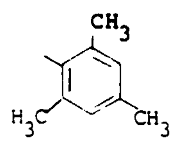 | $-CH_2-$ 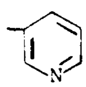 | $(CH_2)_3-$ 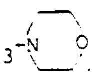 | 1,5(acide fumarique) 0,5$H_2O$ | 81°C |
| 30 | H | 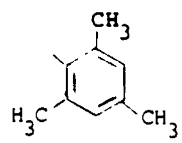 | $-CHCH_3-$ 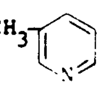 | $-(CH_2)_2-N(C_2H_5)_2$ | 3HCl, 2$H_2O$ | 182-186°C |

TABLEAU 4 (suite 4)

| Ex. | $R_3$ | $Ar_1$ | $-Z_2-Ar_2$ | $-Z_1-W$ | amine ou sel de | F°C |
|---|---|---|---|---|---|---|
| 31 | $-C_5H_{13}$ | 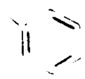 | $-CH_2$ 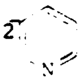 | $-(CH_2)_2-N(CH_3)_2$ | 3HCl, $2H_2O$ | |
| 32 | H | 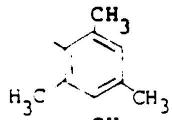 | $-CHCH_3-$ 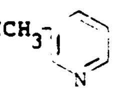 | $-CHCH_3$ 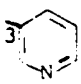 | amine | 168°C |
| 33 | H | 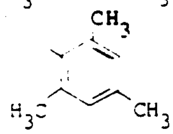 | $-CHCH_3-$ 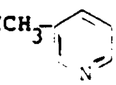 | $-(CH_2)_2-$ 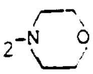 | 1,5(acide fumarique) $H_2O$ | 148°C |
| 34 | H | 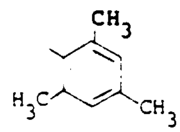 | $-CHCH_3-$ 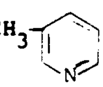 | $-CH_2$ 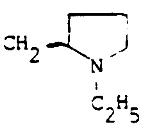 | 2HCl, $H_2O$ | 89°C |
| 35 | H | 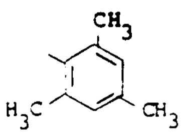 | $-CHCH_3-$ 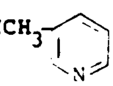 | $-(CH_2)_2-$ 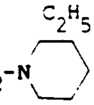 | 3HCl, $2H_2O$ | 100-110°C |
| 36 | H | 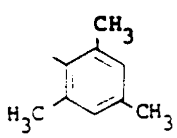 | $-CH_2-$ 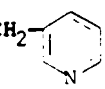 | $-(CH_2)_2-N(i-C_3H_7)_2$ | 3HCl, $1,5H_2O$ | 120°C |
| 37 | H | 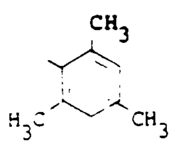 | $-CHCH_3-$ 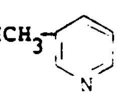 | $-(CH_2)_2-N(i-C_3H_7)_2$ | 3HCl, $2H_2O$ | |
| 38 | H | 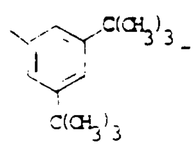 | $-CH_2-$ 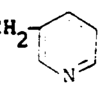 | $-(CH_2)_2-N(CH_3)_2$ | acide fumarique | 164°C |

TABLEAU 4 (suite 5)

| Ex. | $R_3$ | $Ar_1$ | $Z_2$-$Ar_2$ | $-Z_1$-W | amine ou sel de | F°C |
|---|---|---|---|---|---|---|
| 39 | H | 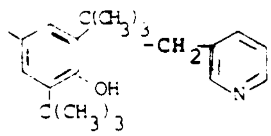 | | $-(CH_2)_2-N(CH_3)_2$ | amine | 140°C |
| 40 | H | 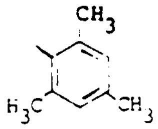 | 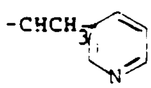 | $-(CH_2)_2-N(C_4H_9)_2$ | 3HBr, 1,5$H_2O$ | 151°C |
| 41 | H | 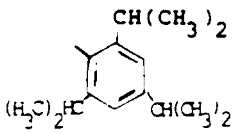 | 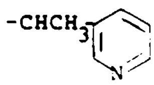 | $-(CH_2)_2-N(CH_3)_2$ | 3HCl, 2$H_2O$ | 140°C |
| 42 | H | 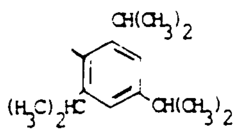 | 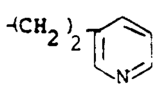 | $-(CH_2)_2-N(CH_3)_2$ | amine | 110°C |
| 43 | H | 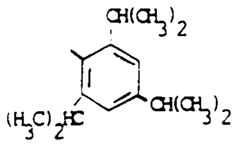 | 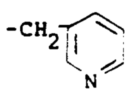 | $-(CH_2)_3-N(CH_3)_2$ | 2 $(COOH)_2$ 0,5$H_2O$ | 144°C |
| 44 | H | 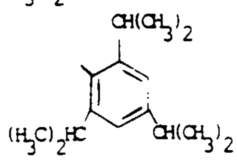 | 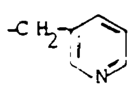 | 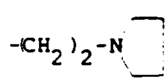 | amine 0,5$H_2O$ | 97°C |
| 45 | H | 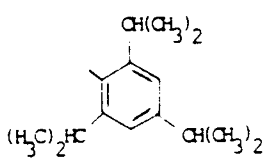 | 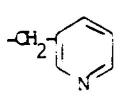 | 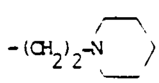 | 2 $(COOH)_2$, 0,5$H_2O$ | 110°C |

TABLEAU 4 (suite 6)

| Ex. | $R_3$ | $Ar_1$ | $Z\text{-}_2\text{-}Ar_2\text{-}$ | $-Z_1\text{-}W\text{-}$ | amine ou sel de | F°C |
|---|---|---|---|---|---|---|
| 46 | H | 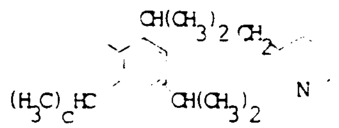 | | 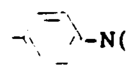 $-N(CH_3)_2$ | $2(COOH)_2$, $1H_2O$ | 156 158 |
| 47 | H | 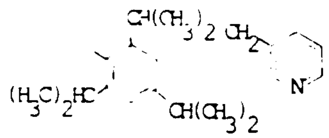 | | $-(CH_2)_4-N(CH_3)_2$ | $2(COOH)_2$, | 148 |
| 48 | H | 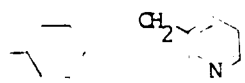 | | $-(CH_2)_2-N(CH_3)_2$ | $2(COOH)_2$, $H_2O$ | 122 |
| 49 | H | 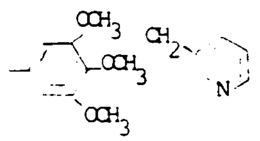 | | $-(CH_2)_2-N(CH_3)_2$ | $2(COOH)_2$ | 152 |
| 50 | $CH_3$ | 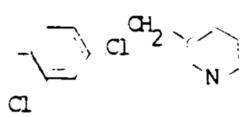 | | $-(CH_2)_2-N(CH_3)_2$ | $(COOH)_2$, 0,5 $H_2O$ | 186 |
| 51 | $CH(CH_3)_2$ | 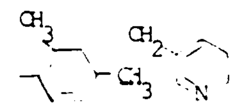 | | $-(CH_2)_2-N(CH_3)_2$ | $2(COOH)_2$, $H_2O$ | 88 |
| 52 | H | 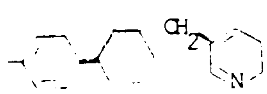 | | $-(CH_2)_2-N(CH_3)_2$ | - | 72 |

TABLEAU 4 (suite 7)

| Ex. | $R_3$ | $Ar_1$ | $Z-_2-Ar_2-$ | $-Z_1-W-$ | amine ou sel de | F°C |
|---|---|---|---|---|---|---|
| 53 | H | 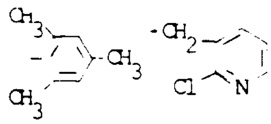 | | $-(CH_2)_2-N(CH_3)_2$ | $1,5(COOH)_2$, 0,75 $H_2O$ | 121 |
| 54 | H | 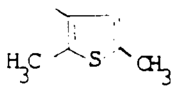 | 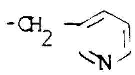 | $-(CH_2)_2-N(CH_3)_2$ | $(COOH)_2$, 0,5 $H_2O$ | 179 |
| 55 | H | 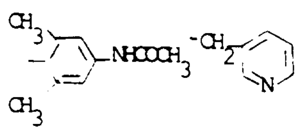 | | $-(CH_2)_2-N(CH_3)_2$ | $2(COOH)_2$, 1,5 $H_2O$ | 98 |
| 56 | H | 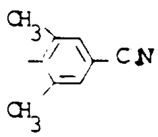 | 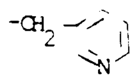 | $-(CH_2)_2-N(CH_3)_2$ | $(COOH)_2$ 1 $H_2O$ | 200 |
| 57 | H | 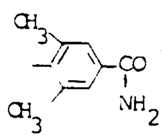 | 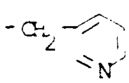 | $-(CH_2)_2-N(CH_3)_2$ | $2(COOH)_2$, $0,5C_3H_7OH$ | 145 |
| 58 | H | 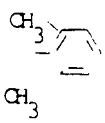 | 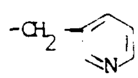 | $-(CH_2)_2-N(CH_3)_2$ | $2(COOH)_2$ | 152 |

TABLEAU 4 (suite 8)

| Ex. | $R_3$ | $Ar_1$ | $Z_2$-$Ar_2$- | -$Z_1$-W- | amine ou sel de | F°C |
|---|---|---|---|---|---|---|
| 59 | H | 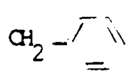 | 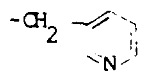 | $-(CH_2)_2-N(CH_3)_2$ | $2(COOH)_2$, 0,5 $H_2O$ | 135 |
| 60 | $CH(CH_3)_2$ | 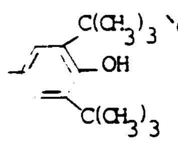 | 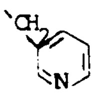 | $-(CH_2)_2-N(CH_3)_2$ | 3HCl, $4H_2O$ | 180-200 |
| 61 | H | 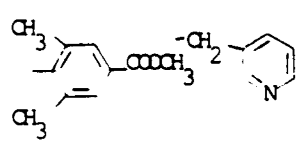 | | $-(CH_2)_2-N(CH_3)_2$ | $2(COOH)_2$ | 130 |
| 62 | H | 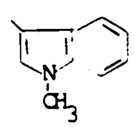 | 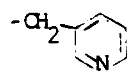 | $-(CH_2)_2-N(CH_3)_2$ | $(COOH)_2$, 0,75 $H_2O$ | 145 |
| 63 | H | 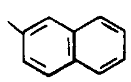 | 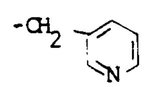 | $-(CH_2)_2-N(CH_3)_2$ | $(COOH)_2$ | 158 |

EXEMPLE 64 : N-[pipéridino-2 éthyl] N-[pyridyl-3 méthyl] amino-2 (triméthyl-2,4,6 phényl)-4 thiazole. (formule I : A = S; B = C; $Ar_1$ = $(CH_3)_3$-2,4,6 $C_6H_2$; $R_3$ = H; $Ar_2$ = pyridyl-3; $Z_1$ = $(CH_2)_2$; $Z_2$ = $CH_2$; W = pipéridino).

On maintient durant 48 heures à 50 °C, 2,3 g de (triméthyl-2,4,6 phényl)-1 thiocyanato-2 éthanone et 2,1 g de N-pipéridino N'-[pyridyl-3 méthyl] éthane diamine dans 40 ml de toluène anhydre. A température ambiante, on extrait par 50 ml d'une solution aqueuse de HCl N la phase organique; après lavage de la phase aqueuse par 2 fois 20 ml de chlorure de méthylène, celle-ci est amenée vers pH 8 par addition d'une solution aqueuse de NaOH 10N, puis extraite par 3 fois 30 ml de chlorure de méthylène. Les phases organiques sont concentrées après séchage et l'huile résiduelle est purifiée par chromatographie sur colonne de silice, en éluant à l'éther éthylique puis avec un mélange chlorure de méthylène/méthanol (96/4,v/v).

Le produit final est une huile dont on prépare un oxalate par action de 0,65 g d'acide oxalique dihydraté sur 1,08 g d'huile dans 35 ml d'acétone. On isole ainsi le sel, monohydraté, qui comporte 2 molécules d'acide pour une du produit de formule I et qui fond à 100 °C.

RMN[1]H(DMSOd6) sel : $\delta$ = 8,53(m,2H) ; 7,75 (m,1H) ; 7,40(m,1H) ; 6,68(s,2H) ; 6,63(s,1H) ; 4,73(m,2H) ; 3,91(m,2H) ; 3,31(m,2H) ; 3,16(m,4H) ; 2,34(s,3H) ; 2,06(s,6H) ; 1,61(m,4H) ; 1,46(m,2H).

EXEMPLE 65 : N-[N'-tertiobutyloxycarbonyl N'-méthyl) amino-2 éthyl] N-[pyridyl-3 méthyl]amino-2 (triisopropyl-2,4,6 phényl)-4 thiazole.
(formule I : A = S; B = C; $Ar_1$ = $[CH(CH_3)_2]_3$-2,4,6 $C_6H_2$; $R_3$ = H; $Ar_2$ = pyridyl-3; $Z_1$ = $(CH_2)_2$; $Z_2$ = $CH_2$; W = $N(CH_3)COOC(CH_3)_2$.

On maintient pendant 72 heures à 65°C, 4,2 g de N-(tertiobutyloxycarbonyl N-méthyl) N'-[pyridyl-3 méthyl] éthanediamine et 4,8 g de (triisopropyl-2,4,6) phényl-1 thiocyanato-2 éthanone dans 50 ml de toluène. On élimine alors le solvant sous pression réduite et purifie l'huile résiduelle par chromatographie sur colonne de silice en éluant tout d'abord par un mélange d'éther éthylique et de chlorure de méthylène (50/50-v/v) et enfin du chlorure de méthylène. On isole ainsi 4,53 g du produit attendu sous forme d'huile.
RMN$^1$H($CDCl_3$) amine :$\delta$ = 8,59(m,2H) ; 7,66 (m,1H) ; 7,25(m,1H) ; 7,03(s,2H) ; 6,27(s,1H) ; 4,77(s,2H) ; 3,50(m,4H) ; 2,87(s,3H) ; 2,9-2,74(m,3H) ; 1,44(s,9H); 1,26(d,6H) ; 1,14(d,12H).

EXEMPLE 66 : N-(N'-méthylamino-2 éthyl) N-[pyridyl-3 méthyl]amino-2 (triisopropyl-2,4,6 phényl)-4 thiazole.
(formule I : A = S; B = C; $Ar_1$ = $[CH(CH_3)_2]_3$-2,4,6 $C_6H_2$; $R_3$ = H; $Ar_2$ = pyridyl-3; $Z_1$ = $(CH_2)_2$; $Z_2$ = $CH_2$; W = $NHCH_3$).

On dissout, sous atmosphère inerte, 1,9 g du composé précédent dans 20 ml d'acétate d'éthyle anhydre avec 5 ml d'anisole et à5°C, on ajoute lentement 20 ml d'une solution de HCl 5N dans l'acétate d'éthyle. On laisse revenir à température ambiante et après 2 heures d'agitation on refroidit vers 5°C avant d'ajouter une solution aqueuse de NaOH 5N jusqu'à pH basique. La phase organique est alors décantée, séchée et concentrée vers 70°C sous pression réduite. L'huile résiduelle est dissoute dans 60 ml d'isopropanol et décolorée par 50 mg de charbon actif et on ajoute 50 ml d'une solution 0,5 M d'acide oxalique dihydraté dans l'isopropanol; le sel précipité est isolé par filtration. On obtient ainsi 1,5 g d'oxalate, comprenant deux molécules d'acide oxalique, hémihydraté, F = 130°C.
RMN$^1$H(DMSOd6) sel :$\delta$ = 8,53(m,2H) ; 7,72(m,1H) ; 7,39(m,1H) ; 7,02(s,2H) ; 6,63(s,1H) ; 4,75(m,2H) ; 3,75(t,2H) ; 3,22(t,2H) ; 2,86(s,1H) ; 2,71(s,2H) ; 2,58(s,3H) ; 1,21(d,6H) ; 1,08(d,12H).

EXEMPLE 67 : N-[(N'-tertiobutyloxycarbonyl)amino-2 éthyl] N-[pyridyl-3 méthyl]amino-2 (triisopropyl-2,4,6 phényl)-4 thiazole.
(formule I : A = S; B = C; $Ar_1$ = $[CH(CH_3)_2]_3$-2,4,6 $C_6H_2$; $R_3$ = H; $Ar_2$ = pyridyl-3; $Z_1$ = $(CH_2)_2$; $Z_2$ = $CH_2$; W = $NHCOOC(CH_3)_3$.)

Ce produit est préparé en appliquant la méthode de l'exemple 65 à partir de N-[tertiobutyloxycarbonyl] N'-[pyridyl-3 méthyl] éthane diamine.
F = 55°C.
RMN$^1$H(DMSOd6) amine :$\delta$ = 8,50(m,2H) ; 7,77(d,1H) ; 7,33(m,1H) ; 7,00(s,3H) ; 6,54(s,1H) ; 4,74(s,2H) ; 3,43(m,2H) ; 3,20(m,2H) ; 2,86(m,1H) ; 2,70(m,2H) ; 1,37(s,9H) ; 1,21(d,6H) ; 1,05(d,12H).

EXEMPLE 68 : N-[amino-2 éthyl] N-[pyridyl-3 méthyl]amino-2 (triisopropyl-2,4,6 phényl)-4 thiazole.
(formule I : A = S; B = C; $Ar_1$ = $(CH(CH_3)_2)_3$-2,4,6 $C_6H_2$; $R_3$ = H; $Ar_2$ = pyridyl-3; $Z_1$ = $(CH_2)_2$; $Z_2$ = $CH_2$; W = $NH_2$).

Composé préparé à partir de celui de l'exemple 67 en appliquant la méthode décrite dans l'exemple 66.
Le dioxalate, hémihydrate fond à 187°C.
RMN$^1$H(DMSOd6) sel :$\delta$ = 8,53(m,2H) ; 8,2(s,2H) ; 7,74(m,1H) ; 7,38(m,1H) ; 7,02(s,2H) ; 6,62(s,1H) ; 4,74-(s,2H) ; 3,71(m,2H) ; 3,10(m,2H) ; 2,89(m,1H) ; 2,71(m,2H) ; 1,21(d,6H) ; 1,08(d,12H).
Les composés de formule I des exemples 69 à 77, décrits dans le tableau 5, pour lesquels A = S et B = C, ont été préparés en appliquant les modes opératoires des exemples 64 à 66 ; les spectres RMN de ces composés figurent dans le tableau 6.

TABLEAU 5

| Ex. | $R_3$ | $-Z_1-W$ | $Z_2-Ar_2$ | $Ar_1$ | amine ou sel de | F°C |
|---|---|---|---|---|---|---|
| 69 | H | 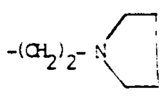 | 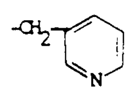 | 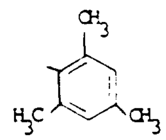 | $3(CF_3COOH)$ | |
| 70 | H | 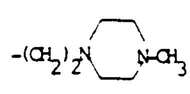 | 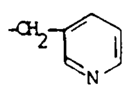 | 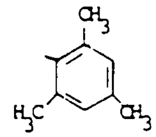 | $2(COOH)_2$ | 202° |
| 71 | H | $-(CH_2)_2-N(CH_3)_2$ | 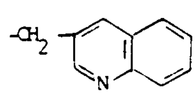 | 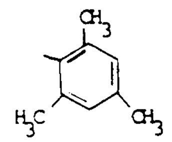 | 1,5(+)-acide tartrique | 110° |
| 72 | H | $-(CH_2)_2-N(CH_3)_2$ | 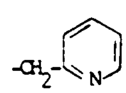 | 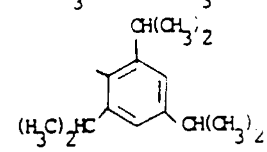 | $2(COOH)_2$ | 164° |
| 73 | H | $-(CH_2)_2-N(CH_3)_2$ | 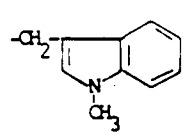 | 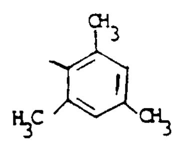 | $(COOH)_2$, $H_2O$ | 155° |
| 74 | H | $-(CH_2)_2SC_2H_5$ | 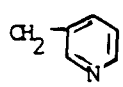 | 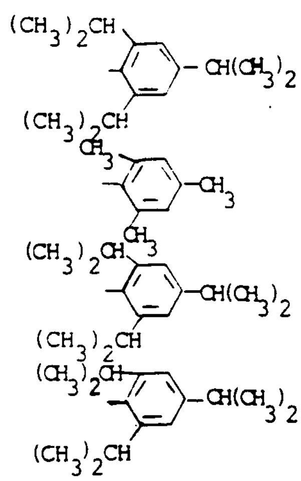 | 2HCl, $H_2O$ | 145 |
| 75 | H | $-CH_2CON(CH_3)_2$ | 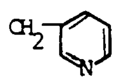 | | $2H_3PO_4$ $C_3H_7OH$ | 140 |
| 76 | H | $-CH_2COOCH_3$ | 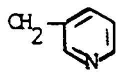 | | - | huile |
| 77 | H | $-(CH_2)_2OCH_3$ | 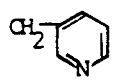 | | - | 94 |

EXEMPLE 78 : N-[(N'-méthyl N'-phényl amino)-2 éthyl] N-[pyridyl-3 méthyl]amino-2 (triméthyl-2,4,6) phényl-4 thiazole.

(formule I : A = S; B = C; $Ar_1$ = $(CH_3)_3$-2,4,6 $C_6H_2$; $R_3$ = H; $Ar_2$ = pyridyl-3; $Z_1$ = $(CH_2)_2$; $Z_2$ = $CH_2$; W = $NCH_3C_6H_5$).

On maintient vers 60 °C pendant 96 heures environ, sous atmosphère inerte, 1 g de N-phényl N-méthyl N'-(pyridyl-3 méthyl) éthanediamine, et 1,5 g de bromhydrate de bromo-2 (triméthyl-2,4,6 phényl)-4 thiazole dans 50 ml de toluène. On concentre alors sous pression réduite et verse sur le résidu 20 ml d'une solution

aqueuse de NaOH N avant d'extraire par 3 fois 20 ml de chlorure de méthylène. Les phases organiques, lavées et séchées, sont concentrées sous pression réduite et l'huile résiduelle est purifiée par chromatographie sur colonne de silice en éluant avec un mélange de chlorure de méthylène et de méthanol (99/1-v/v). On isole ainsi 1,1 g de produit final sous forme d'huile.

le trichlorhydrate, préparé par action de HCl dans l'éther éthylique, cristallise avec 1,5 molécule d'eau ; il fond à 160 °C.

RMN[1]H(DMSOd6) sel :δ = 8,84(m,2H) ; 8,45(m,1H) ; 7,97(m,1H) ; 7,15(m,2H) ; 6,85(m,5H) ; 6,67(s,1H) ; 4,92(s,2H) ; 3,75(m,4H) ; 2,92(s,3H) ; 2,22(s,3H) ; 1,98(s,6H).

<u>EXEMPLE 79</u> : N-[N',N'-diméthylamino-2 éthyl] N-[pyridyl-3 méthyl]amino-2 (dichloro-2,4 phényl)-4 méthyl-5 oxazole.
(formule I : A = O; B = C; $Ar_1$ = $(Cl)_2$-2,4 $C_6H_3$; $R_3$ = $CH_3$; $Ar_2$ = pyridyl-3; $Z_1$ = $(CH_2)_2$; $Z_2$ = $CH_2$; W = $N(CH_3)_2$).

Une solution de 0,7 g de chloro-2 (dichloro-2,4 phényl-4 méthyl-5 oxazole et 1,4 g de N-[diméthylamino-2 éthyl] N-[pyridyl-3 méthyl]amine dans 40 ml de toluène anhydre est maintenue 80 heures vers 95 °C, sous atmosphère inerte. Le solvant est alors évaporé sous pression réduite, on verse sur le résidu 3 ml de méthanol et une solution saturée de bicarbonate de sodium jusqu'à pH basique pour libérer l'amine. On concentre ensuite sous pression réduite, et le résidu est purifié par chromatographie sur une colonne de silice en éluant successivement avec de l'éther éthylique, du chlorure de méthylène et un mélange chlorure de méthylène/méthanol (98/2-v/v).

On isole ainsi 0,45 g du produit attendu sous forme d'huile.

Son trihémioxalate, [1,5$(COOH)_2$] préparé par action d'acide oxalique hydraté dans l'isopropanol, cristallise avec $3H_2O$. F = 130 °C.

RMN[1]H(DMSOd6) sel :δ = 8,60(s,1H) ; 8,53(m,1H) ; 7,78(m,2H) ; 7,44(m,3H) ; 4,64(s,2H) ; 3,7(t,2H) ; 3,36-(t,2H) ; 2,82(s,6H) ; 2,2(s,3H) ;

<u>EXEMPLE 80</u> : N-[N',N'-diméthylamino-2 éthyl] N-[pyridyl-3 méthyl]amino-2 (méthyl-4 phényl)-4 méthyl-5 oxazole.
(formule I : A = O; B = C; $Ar_1$ = $(CH_3)$-4 $C_6H_4$; $R_3$ = $CH_3$; $Ar_2$ = pyridyl-3; $Z_1$ = $(CH_2)_2$; $Z_2$ = $CH_2$; W = $N(CH_3)_2$).

Ce composé a été préparé en appliquant le procédé de l'exemple 79.

Le dioxalate, [2$(COOH)_2$] cristallisé avec 1,5 $H_2O$ fond à 180-190 °C.

RMN[1]H(DMSOd6) base :δ = 8,55(m,2H) ; 7,70(m,1H) ; 7,45(m,3H) ; 7,22(m,2H) ; 4,66(s,2H) ; 3,76(m,2H) ; 3,38(m,2H) ; 2,86(s,6H) ; 2,43(s,3H) ; 2,32(s,3H).

<u>EXEMPLE 81</u> : N-[pyrrolidino-2 éthyl] n-[pyridyl-3 méthyl]amino-2 (dichloro-2,4 phényl)-4 méthyl-5 oxazole.
(Formule I : A = O; B = C; $AR_1$ = $(CL)_2$-2,4 $C_6H_3$; $R_3$ = $CH_3$; $Ar_2$ = pyridyl-3; $Z_1$ = $(CH_2)_2$; $Z_2$ = $CH_2$;

W = 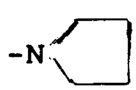 ).

Ce composé a été préparé en appliquant le procédé de l'exemple 79.

L'oxalate, cristallisé avec 4 $H_2O$, fond à 145 °C.

RMN[1]H(DMSOd6) sel :δ = 8,5(m,2H) ; 7,8(m,2H) ; 7,4(m,3H) ; 4,6(s,2H) ; 4-3(m,8H) ; 2,2(s,3H) ; 1,9(m,4H).

<u>EXEMPLE 82</u> : N-[N',N'-diméthylamino-2 éthyl] N-[(pyridyl-3)-2 éthyl]amino-5 (triméthyl-2,4,6 phényl)-3 thiadiazole-1,2,4.
(formule I : A = S; B = N; $Ar_1$ = $(CH_3)_3$-2,4,6 $C_6H_2$; $R_3$ = rien; $Ar_2$ = pyridyl-3; $Z_1$ = $(CH_2)_2$; $Z_2$ = $CH_2$; W = $N(CH_3)_2$).

On maintient 7 heures vers 55 °C sous atmosphère inerte 0,45 g de chloro-5 (triméthyl-2,4,6 phényl)-3 thiadiazole-1,2,4 et 1,2 g de N,N-diméthyl N'-((pyridyl-3)-2 éthyl)éthane-diamine en solution dans 10 ml d'éthanol, puis à tempéature ambiante, on ajoute 0,5 ml d'une solution aqueuse de NaOH 1N et 2 g de silice. On concentre à siccité et chromatographie le résidu sur colonne de silice, en éluant successivement

avec du dichlorométhane pur puis en mélange avec du méthanol (98/2-v/v); On obtient ainsi 0,52 g du produit cherché sous forme d'huile.

Le dioxalate, préparé dans l'acétone, cristallisé avec 1$H_2O$, fond à 131 °C.

RMN[1]H(DMSOd6) sel :δ = 8,45(m,2H) ; 7,7(m,1H) ; 7,3(m,1H) ; 6,9(s,2H) ; 3,9(m,2H) ; 3,6(m,2H) ; 3,3(m,2H) ; 3(m,2H) ; 2,8(s,6H) ; 2,27(s,3H) ; 2,09(m,6H).

Les composés des exemples 83 à 86 ont été préparés en appliquant le procédé de l'exemple 82.

EXEMPLE 83 : N-[N',N'-diméthylamino-2 éthyl] N-[pyridyl-3 méthyl]amino-5 (triméthyl-2,4,6 phényl)-3 thiadiazole-1,2,4.
(formule I : A = S; B = N; $Ar_1$ = $(CH_3)_3$-2,4,6 $C_6H_2$; $R_3$ = rien; $Ar_2$ = pyridyl-3; $Z_1$ = $(CH_2)_2$; $Z_2$ = $CH_2$; W = $N(CH_3)_2$).

Le sel avec 1,5 molécule d'acide oxalique, cristallisé avec 0,5 molécule d'eau et 0,5 molécule d'acétone, fond vers 145 °C.

EXEMPLE 84 : N-[N',N'-diméthylamino-2 éthyl] N-[pyridyl-3 méthyl]amino-5 phényl-3 thiadiazole-1,2,4.
(formule I : A = S; B = N; $Ar_1$ = $C_6H_5$; $R_3$ = rien; $Ar_2$ = pyridyl-3; $Z_1$ = $(CH_2)_2$; $Z_2$ = $CH_2$; W = N-$(CH_3)_2$).

Le dioxalate de ce composé fond à 173 °C.

EXEMPLE 85 : N-[méthoxyéthyl] N-[pyridyl-3 méthyl]amino-5 (triméthyl-2,4,6 phényl)-3thiadiazole-1,2,4.
(formule I : A = S; B = N; $Ar_1$ = $(CH_3)_3$-2,4,6 $C_6H_2$; $R_3$ = rien; $Ar_2$ = pyridyl-3; $Z_1$ = $(CH_2)_2$; $Z_2$ = $CH_2$; W = $OCH_3$).

Le dioxalate de ce composé, cristallisé avec 1,5 $H_2O$, fond à 145 °C.

EXEMPLE 86 : N-[N',N'-diméthylamino-2 éthyl] N-[pyridyl-3 méthyl]amino-5 (diméthyl-2,4 phényl)-3 oxadiazole-1,2,4.
(formule I : A = O; B = N; $Ar_1$ = $(CH_3)_2$-2,4$C_6H_3$ ; $R_3$ = rien; $Ar_2$ = pyridyl-3; $Z_1$ = $(CH_2)_2$; $Z_2$ = $CH_2$; W = $N(CH_3)_2$).

On maintient sous agitation, en atmosphère inerte, pendant 16 heures, à température ambiante, 0,5 g de chloro-5 (diméthyl-2,4 phényl)-3 oxadiazole-1,2,4 et 1,3 g de N,N-diméthyl N'-[pyridyl-3 méthyl]éthane diamine dans 20 ml de toluène anhydre. On élimine le précipité par filtration et on ajoute au filtrat 10 ml d'une solution d'ammoniaque à 5% (p/v) ; la phase organique est séparée, et la phase réextraite par 2 fois avec 15 ml d'acétate d'éthyle. Les phases organiques réunies sont concentrées sous pression réduite et le résidu est purifié par chromatographie sur une colonne de silice en éluant avec un mélange de chlorure de méthylène et de méthanol (98/2-v/v). On obtient ainsi 0,6 g du produit attendu sous forme d'huile.

Son dioxalate, préparé dans l'acétone, fond à 166 °C.

RMN[1]H(DMSOd6) sel : 8,62(m,1H) ; 8,53(m,1H) ; 7,7(m,2H) ; 7,4(m,1H) ; 7,16(m,2H) ; 4,77(s,2H) ; 3,9(t,2H) ; 3,4(t,2H) ; 2,82(s,6H) ; 2,47(s,3H). 2,31 (s,3H).

EXEMPLE 87 : N-[N',N'-diméthylamino-2 éthyl] N-[pyridyl-2 méthyl]amino-5 (diméthyl-2,4 phényl)-3 oxadiazole-1,2,4.
(formule I : A = O; B = N; $Ar_1$ = $(CH_3)_2$-$C_6H_3$ ; $R_3$ = rien; $Ar_2$ = pyridyl-2; $Z_1$ = $(CH_2)_2$; $Z_2$ = $CH_2$; W = $N(CH_3)_2$).

Préparé en appliquant le mode opératoire décrit à l'exemple 86.

Le sel d'acide oxalique, qui comprend 2,5 molécules d'acide oxalique fond à 148 °C.

RMN[1]H(DMSOd6) sel :δ = 8,57(d,1H) ; 7,88(t,1H) ; 7,71(d,1H) ; 7,49(d,1H) ; 7,4(m,1H) ; 7,14(m,2H) ; 4,84-(s,2H) ; 4,0(t,2H) ; 3,48(t,2H) ; 2,9(s,6H); 2,43(s,3H) ; 2,3(s,3H).

EXEMPLE 88 : N-[N',N'-diméthylamino-2 éthyl] N-[pyridyl-3 méthyl]amino-5 phényl-3 oxadiazole-1,2,4.
(formule I : A = O; B = N; $Ar_1$ = $C_6H_5$; $R_3$ = rien; $Ar_2$ = pyridyl-3; $Z_1$ = $(CH_2)_2$; $Z_2$ = $CH_2$; W = N-$(CH_3)_2$).

i- N-[N',N'-diméthylamino-2 éthyl]amino-5 phényl-3 oxadiazole-1,2,4.

On maintient à 35 °C environ 65 heures, un mélange de 1,5 g de trichlorométhyl-5 phényl-3 oxadiazole-1,2,4, préparé comme décrit dans Helv. chem. Acta 46 1067-1073 (1963), et 2,2 g de N,N-diméthyléthane diamine. L'amine en excès est éliminée par distillation sous pression réduite et on verse sur le résidu 10 ml d'une solution aqueuse saturée de bicarbonate de sodium; la phase aqueuse est extraite par 3 fois avec 10 ml d'acétate d'éthyle et les phases organiques réunies sont concentrées à siccité, après lavage à l'eau et séchage.

On isole ainsi 1,32 g du produit attendu sous forme d'huile.

ii- N-[N',N'-diméthylamino-2 éthyl] N-[pyridyl-3 méthyl]amino-5 phényl-3 oxadiazole-1,2,4.

On maintient sous agitation durant 16 heures, un mélange de 1 g du produit obtenu en i/, 0,78 g de chlorométhyl-3 pyridine, 25 ml de chlorure de méthylène et 20 ml d'une solution aqueuse de NaOH à 50% (p/v).

La phase organique est alors séparée et la phase aqueuse réextraite par un volume de chlorure de méthylène. Les phases organiques réunies sont lavées à l'eau, séchées et concentrées. Le résidu est chromatographié sur silice en éluant successivement avec de l'éther éthylique, du chlorure de méthylène et un mélange de chlorure de méthylène/méthanol (97/3-v/v); On obtient ainsi 0,8 g du produit cherché.

Le dioxalate ($2(COOH)_2$) préparé dans l'acétone, cristallisé avec 0,5 $H_2O$ fond à 125 °C.

RMN$^1$H(DMSOd6) sel :$\delta$ = 8,65(s,1H) ; 8,56(m,1H) ; 7,9(m,3H) ; 7,5(m,4H) ; 4,80(s,2H) ; 3,93(t,2H) ; 3,42-(t,2H) ; 2,86(s,6H).

EXEMPLE 89 : N-oxyde du composé de l'exemple 2.

(formule I : A = S; B = C; $Ar_1$ = $[CH(CH_3)_2]_3$-2,4,6 $C_6H_2$; $R_3$ = H; $Ar_2$ = pyridyl-3; $Z_1$ = $(CH_2)_2$; $Z_2$ = $CH_2$; W = $N(O)(CH_3)_2$.

On introduit 0,5 g du composé de l'exemple 2 dans 5 ml d'une solution dans le chloroforme de 0,56 g de (phénylsulfonyl)-2 phényl-3 oxaziridine, préparé comme décrit dans Org. Synth. 66 203-210 (1987). Après 1 heure d'agitation à température ambiante, sous atmosphère inerte, on concentre le milieu sous pression réduite et on chromatographie le résidu sur une colonne de silice en éluant successivement avec un mélange de chlorure de méthylène/méthanol (95/5 puis 90/10 v/v) et chlorure de méthylène/méthanol/$NH_4OH$ aqueux à 25% (p/v) (80/15/5-v/v).

On obtient ainsi 0,35 g du N-oxyde, cristallisé avec 2 molécules d'eau qui fond à 115 °C.

RMN$^1$H(DMSOd6) base :$\delta$ = 8,56(d,1H) ; 8,55(m,1H) ; 7,73(m,1H) ; 7,35(m,1H) ; 7,01(s,2H) ; 6,59(s,1H) ; 4,79(s,2H) ; 3,97(m,2H) ; 3,47(m,2H) ; 3,05(s,6H) ; 2,87(m,1H) ; 2,7(m,2H) ; 1,21(d,6H) ; 1,07(d,12H).

EXEMPLE 90 : N-[N',N'-diméthylamino-2 éthyl] N-[pyridyl-3 méthyl]amino-2 bromo-5 (triisopropyl-2,4,6 phényl)-4 thiazole.

(formule I : A = S; B = C; $Ar_1$ = $[CH(CH_3)_2]_3$-2,4,6 $C_6H_2$; $R_3$ = Br; $Ar_2$ = pyridyl-3; $Z_1$ = $(CH_2)_2$; $Z_2$ = $CH_2$; W = $N(CH_3)_2$).

On dissout 2,3 g du composé de l'exemple 2 dans 100 ml d'une solution aqueuse d'acide bromhydrique 0,05 N, introduit lentement 0,8 g de brome et porte le mélange au reflux 30 minutes, avant de concentrer à siccité sous pression réduite. Le résidu, dissout dans un mélange chlorurede méthylène/méthanol (95/5-v/v) est filtré sur silice puis concentré.

Le solide résiduel est précipité dans l'éthanol par addition d'éther éthylique.

On obtient ainsi 1,2 g du dibromhydrate monohydraté du composé cherché qui se sublime vers 250 °C.

RMN$^1$H(DMSOd6) sel :$\delta$ = 8,84(m,2H) ; 8,30(m,1H) ; 7,94(m,1H) ; 7,05(s,2H) ; 4,91(s,2H) ; 3,94(t,2H) ; 3,47-(t,2H) ; 2,86(s,6H) ; 2,52(m,3H) ; 1,24(d,6H) ; 1,13(d,6H) ; 1,01(d,6H).

EXEMPLE 91 : N-[N',N'-diméthylamino-2 éthyl] N-[pyridyl-3 méthyl]amino-2 chloro-5 (triisopropyl-2,4,6 phényl)-4 thiazole.

(formule I : A = S; B = C; $Ar_1$ = $[CH(CH_3)_2]_3$-2,4,6 $C_6H_2$; $R_3$ = Cl; $Ar_2$ = pyridyl-3; $Z_1$ = $(CH_2)_2$; $Z_2$ = $CH_2$; W = $N(CH_3)_2$).

On dissout 2,3 g du composé de l'exemple 2 dans un mélange de 80 ml d'eau et 10 ml d'une solution aqueuse de HCl N et introduit dans la solution 0,36 g de chlore gazeux. Après une nuit à température ambiante on concentre à siccité, et on verse sur le résidu 20 ml environ d'une solution aqueuse de NaOH 2N, glacée, jusqu'à pH nettement basique. La phase aqueuse est extraite par le chlorure de méthylène; la phase organique après lavage et séchage est concentré et le résidu est purifié par chromatograpgie sur silice en éluant avec un mélange de chlorure de méthylène et méthanol (98/2-v/v).

Le dioxalate, préparé dans l'acétone, fond à 178 °C.
RMN[1]H(DMSOd6) sel :δ = 8,54(m,2H) ; 7,70(m,1H) ; 7,40(m,1H) ; 7,06(s,2H) ; 4,71(s,2H) ; 3,82(t,2H) ; 3,30-(t,2H) ; 2,76(s,6H) ; 2,61(m,3H) ; 1,24(d,6H) ; 1,13(d,6H) ; 1,04(d,6H).

EXEMPLE 92 : N-[N',N'-diméthylamino-2 éthyl] N-[pyridyl-3 méthyl]amino-2 (carboxy-4 diméthyl-2,6 phényl)-4 thiazole.
(formule I : A = S; B = C; $Ar_1$ = (COOH)-4$(CH_3)_2$-2,6 $C_6H_2$; $R_3$ = H; $Ar_2$ = pyridyl-3; $Z_1$ = $(CH_2)_2$; $Z_2$ = $CH_2$; W = $N(CH_3)_2$).

On chauffe 3 heures au reflux une solution dans 50 ml de méthanol de 1,6 g du composé de l'exemple 61, avec une solution de 2,13 g de KOH dans 50 ml d'eau: vers 15 °C, on neutralise le milieu par addition d'une solution auquese de HCl 2N puis on évapore le méthanol sous pression réduite. La phase aqueuse est extraite au chlorurede méthylène pourdonner 1,4 g du composé cherché, crisallisé avec 0,5 $H_2O$, qui fond à 125 °C.
RMN[1]H(DMSOd6-$D_2O$) base :δ = 8,53(m,2H) ; 7,72(m,1H) ; 7,60(s,2H) ; 7,35(m,1H) ; 6,60(s,1H) ; 4,72(s,2H) ; 3,55(t,2H) ; 2,47(t,2H) ; 2,15(s,6H) ; 2,10(s,6H).

EXEMPLE 93 : N-méthyl N-[N'-[pyridyl-3 méthyl] N'-[(triisopropyl-2,4,6 phényl)-4 thiazolyl-2]amino-2 éthyl]-acétamide.
(formule I : A = S; B = C; $Ar_1$ = $[CH(CH_3)_2]_3$-2,4,6 $C_6H_2$; $R_3$ = H; $Ar_2$ = pyridyl-3; $Z_1$ = $(CH_2)_2$; $Z_2$ = $CH_2$; W = $NCH_3COCH_3$.

On introduit vers 10 °C dans une solution contenant 0,35 g du composé de l'exemple 66 et 0,2 ml de triéthylamine dans 20 ml de dichlorométhane, 0,06 g de chlorure d'acétyle. Après une heure à température ambiante, on ajoute 20 ml d'eau et sépare la phase organique d'où l'on extrait 0,32 g du composé attendu sous forme d'huile. Le diphosphate, préparé dans l'isopropanol, cristallisé avec 1,5 $H_2O$, fond à 165 °C.
RMN[1]H(DMSOd6) base :δ = 8,5(m,2H) ; 7,7(m,1H) ; 7,4(m,1H) ; 7(s,2H) ; 6,55(m,1H) ; 4,73(s,2H) ; 3,55-(m,4H) ; 3-2,65(m,6H) ; 1,90(s,3H) ; 1,22(d,6H) ; 1,05(d,12H).

EXEMPLE 94 : N-méthyl N-[N'[pyridyl-3 méthyl] N'[(triisopropyl-2,4,6 phényl-4 thiazolyl-2]amino-2 éthyl] méthane sulfonamide.
(formule I : A = S; B = C; $Ar_1$ = $[CH(CH_3)_2]_3$-2,4,6 $C_6H_2$; $R_3$ = H; $Ar_2$ = pyridyl-3; $Z_1$ = $(CH_2)_2$; $Z_2$ = $CH_2$; W = $NCH_3SO_2CH_3$).

Préparé en appliquant le procédé de l'exemple 93 avec du chlorure de méthane sulfonyle au lieu de chlorure d'acétyle.
Ce composé cristallisé avec 1/3 de molécule d'eau fond à 120 °C.
RMN[1]H(DMSOd6) base :δ = 8,55(m,2H) ; 7,72(m,1H) ; 7,30(m,1H) ; 7,02(s,2H) ; 6,28(s,1H) ; 4,78(s,2H) ; 3,69(t,2H) ; 3,40(t,2H) ; 2,86(s,3H) ; 2,80(m,3H) ; 2,76(s,3H) ; 1,26(d,6H) ; 1,14(d,12H).

EXEMPLE 95 : N-méthyl N'-méthyl N'-[N''[pyridyl-3 méthyl] N''[(triisopropyl-2,4,6 phényl)-4 thiazolyl-2]-amino-2 éthyl] thiourée.
(formule I : A = S; B = C; $Ar_1$ = $/CH(CH_3)_2/_3$-2,4,6 $C_6H_2$; $R_3$ = H; $Ar_2$ = pyridyl-3; $Z_1$ = $(CH_2)_2$; $Z_2$ = $CH_2$; W = $NCH_3CSNHCH_3$).

On introduit 0,08 g d'isothiocyanate de méthyle dans 5 ml d'une solution de 0,5 g du composé de l'exemple 66 dans le dichlorométhane. Après une heure d'agitation à température ambiante, on concentre à siccité et on ajoute sur le résidu 10 ml d'éther isopropylique. Le précipité est isolé et recristallisé dans l'acétate d'éthyle, pour donner 0,26 g du composé cherché qui fond à 160 °C.
RMN[1]H($CDCl_3$) base :δ = 8,63(m,2H) ; 7,90(s,1H) ; 7,70(m,1H) ; 7,38(m,1H) ; 7,04(s,2H) ; 6,35(s,1H) ; 4,61-(s,2H) ; 3,55(s,4H) ; 3,26(s,3H) ; 2,90(m,1H) ; 2,70(m,2H) ; 2,07(s,3H) ; 1,22(d,6H) ; 1,11(d,12H).

EXEMPLE 96 : N-méthyl N'-méthyl N'-[N''-[pyridyl-3 méthyl] N''-[(triisopropyl-2,4,6 phényl)-4 thiazolyl-2]-amino-2 éthyl] urée.
(formule I : A = S; B = C; $Ar_1$ = $[CH(CH_3)_2]_3$-2,4,6 $C_6H_2$: $R_3$ = H; $Ar_2$ = pyridyl-3; $Z_1$ = $(CH_2)_2$; $Z_2$ = $CH_2$; W = $NCH_3CONHCH_3$).

Ce composé est préparé en appliquant le procédé de l'exemple 95, en remplaçant l'isothiocyanate de méthyle par de l'isocyanate de méthyle. Il fond à 138 °C.
RMN$^1$H(DMSOd$_6$) base :δ = 8,51(m,2H) ; 7,69(m,1H) ; 7,35(m,1H) ; 7,02(s,2H) ; 6,58(s,1H) ; 6,48(d,1H) ; 4,71(s,2H) ; 3,55(m,2H) ; 3,34(m,2H) ; 2,72(m,3H) ; 2,69(s,3H) ; 2,15(d,3H) ; 1,18(d,6H) ; 1,05(d,12H).

EXEMPLE 97 : N-[N',N'-diméthylamino-2 éthyl] N-[pyridyl-3 méthyl]amino-2 (amino-4 diméthyl-2,6 phényl)-4 thiazole.
(formule I : A = S; B = C; $Ar_1$ = ($NH_2$-4($CH_3$)$_2$-2,6) $C_6H_2$; $R_3$ = H; $Ar_2$ = pyridyl-3; $Z_1$ = $(CH_2)_2$; $Z_2$ = $CH_2$; W = $N(CH_3)_2$).

On porte au reflux durant 4 heures une solution de 0,8 g du composé de l'exemple 55 dans un mélange de 5 ml d'éthanol et 2 ml de solution aqueuse d'acide chlorhydrique 12N. On élimine ensuite les solvants, passe en milieu basique par addition d'une solution aqueuse glacée de NaOH 2N et extrait par l'acétate d'éthyle. L'huile obtenue par évaporation du solvant organique est dissoute dans l'acétone, d'où on précipite le trioxalate du produit cherché par addition d'une solution de 0,3 g d'acide oxalique (dihydrate). On obtient ainsi 0,6 g de produit qui fond à 157 °C.
RMN$^1$H(DMSOd$_6$) sel :δ = 8,55(m,2H) ; 7,73(m,1H) ; 7,41(m,1H) ; 6,51(s,1H) ; 6,29(s,2H) ; 4,72(s,2H) ; 3,86-(t,2H) ; 3,37(t,2H) ; 2,81(s,6H) ; 1,96(s,6H).

TABLEAU 6 : RMN $^1H$ - 250 MHz

| Exemple N° (amine ou sel) | δ(ppm) |
|---|---|
| 3 sel | DMSOd6 : 8,6(s,1H) ; 8,55(m,1H) ; 7,97(m,1H) ; 7,68(m,1H) ; 7,66(s,1H) ; 7,55-7,36(m,3H) ; 4,74(s,2H) ; 3,98(m,2H) ; 3,41(m,2H) ; 2,84(s,6H). |
| 4 sel | DMSOd6 : 8,6(s,1H) ; 8,52(m,1H) ; 7,80(m,1H) ; 7,53(m,2H) ; 7,43(m,2H) ; 6,92(s,1H) ; 4,74(s,2H) ; 3,90(t,2H) ; 3,39(t,2H) ; 2,62(s,6H). |
| 5 sel | DMSOd6 : 8,63(m,2H) ; 7,8-7,4(m,6H) ; 7,31(s,1H) ; 4,69(s,2H) ; 3,86(t,2H) ; 3,34(t,2H) ; 2,83(s,6H). |
| 6 sel | DMSOd6 : 8,5(m,2H) ; 7,8(m,3H) ; 7,3(m,5H) ; 4,7(s,2H) ; 4(t,2H) ; 3,45(t,2H) ; 2,9(s,6H). |
| 7 sel | DMSOd6 : 8,54(m,2H) ; 7,82-7,58(m,5H) ; 7,43(m,1H) ; 6,94(s,1H) ; 4,75(s,2H) ; 3,93(t,2H) ; 3,40(t,2H) ; 2,82(s,6H). |
| 8 sel | DMSOd6 : 8,53(m,2H) ; 7,75(m,1H) ; 7,64(m,2H) ; 7,41(m,3H) ; 4,67(s,2H) ; 3,91(t,2H) ; 3,39(t,2H) ; 2,84(s,6H) ; 2,36(s,3H). |
| 9 sel | DMSOd6 : 8,59(m,1H) ; 8,52(m,1H) ; 7,74(m,1H) ; 7,51(m,2H) ; 7,42(m,1H) ; 7,22(m,2H) ; 4,67(s,2H) ; 3,89(t,2H) ; 3,39(t,2H) ; 2,84(s,6H) ; 2,35(s,3H) ; 2,33(s,3H). |
| 10 amine | DMSOd6 : 8,6(m,2H) ; 7,83(m,1H) ; 7,38(m,1H) ; 7,28(t,1H) ; 6,67(d,2H) ; 6,48(s,1H) ; 4,70(s,2H) ; 3,68(s,6H) ; 3,48(t,2H) ; 2,49(m,2H) ; 2,16(s,6H). |

TABLEAU 6 (suite 1)

| Exemple N° (amine ou sel) | δ(ppm) |
|---|---|
| 11 sel | DMSOd6 : 8,5(m,2H) ; 7,25(m,2H) ; 6,85(s,2H) ; 6,6(s,1H) ; 4,7(s,2H) ; 3,9(t,2H) ; 3,3(t,2H) ; 2,7(s,6H) ; 2,2(s,3H) ; 2(s,6H). |
| 12 amine | DMSOd6 : 8,6(s,1H) ; 8,47(m,1H) ; 7,96(m,1H) ; 7,75(m,1H) ; 7,35(m,1H) ; 7,25-7,1(m,3H) ; 4,72(s,2H) ; 3,55(m,2H) ; 2,9-2,7(m,4H) ; 2,48(m,2H) ; 2,17(s,6H) ; 1,95(m,2H). |
| 13 sel | DMSOd6 : 8,56(s,1H) ; 8,51(m,1H) ; 7,72(m,1H) ; 7,40(m,1H) ; 7,05(m,3H) ; 4,67(s,2H) ; 3,84(m,2H) ; 3,32(m,2H) ; 2,77(s,6H) ; 2,28(s,3H) ; 2,18(s,3H) ; 2,08(s,3H). |
| 14 amine | DMSOd6 : 8,55(s,1H) ; 8,47(m,1H) ; 7,71(m,1H) ; 7,33(m,1H) ; 6,85(s,2H) ; 6,48(s,1H) ; 4,73(s,2H) ; 3,54(m,2H) ; 2,47(m,2H) ; 2,22(s,3H) ; 2,15(s,6H) ; 2,03(s,6H). |
| 15 sel | DMSOd6 : 8,56(s,1H) ; 8,48(m,1H) ; 7,73(m,1H) ; 7,35(m,1H) ; 6,68(s,1H) ; 6,63(s,1H) ; 6,58(s,2H) ; 6,51(s,1H) ; 4,70(s,2H) ; 3,66(s,3H) ; 3,63(m,2H) ; 2,73(m,2H) ; 2,34(s,6H) ; 2,27(s,3H) ; 2,24(s,3H). |
| 16 sel | DMSOd6 : 8,63(s,1H) ; 8,50(m,1H) ; 7,80(m,1H) ; 7,38(m,1H) ; 6,57(s,2H) ; 6,46(s,1H) ; 6,26(s,2H) ; 4,69(s,2H) ; 3,80(s,3H) ; 3,68(s,6H) ; 3,63(m,2H) ; 2,80(m,2H) ; 2,40(s,6H). |

TABLEAU 6 (suite 2)

| Exemple N° (amine ou sel) | δ(ppm) |
|---|---|
| 17 sel | DMSOd6 : 8,95(s,1H) ; 8,90(m,1H) ; 8,55(m,1H) ; 8,05(m,1H) ; 6,88(s,2H) ; 6,77(s,1H) ; 5,07(s,2H) ; 3,75(t,2H) ; 3,1(m,2H) ; 2,72(s,6H) ; 2,23(s,3H) ; 2,15(m,2H) ; 2,02(s,6H). |
| 18 sel | DMSOd6 : 8,9(m,2H) ; 8,6(m,1H) ; 8,1(m,1H) ; 6,9(s,2H) ; 6,7(s,1H) ; 5,45(q,1H) ; 3,9(t,2H) ; 3,3(t,2H) ; 2,8(s,6H) ; 2,25(s,3H) ; 2,0(s,6H) ; 1,8(d,3H). |
| 19 sel | DMSOd6 : 9(m,2H) ; 8,1(m,2H) ; 6,9(s,2H) ; 6,8(s,1H) ; 5,6(m,1H) ; 4,1(t,2H) ; 3,5(m,2H) ; 2,9(s,6H) ; 2,3(s,3H) ; 2(s,6H) ; 1,9(d,3H). |
| 20 sel | DMSOd6 : 8,57(s,1H) ; 8,44(m,1H) ; 7,75(m,1H) ; 7,33(m,1H) ; 6,84(s,2H) ; 6,61(s,4H) ; 6,49(s,1H) ; 4,63(s,2H) ; 4,36(q,1H) ; 2,83(m,1H) ; 2,47(m,1H) ; 2,29(s,6H) ; 2,22(s,3H) ; 1,99(s,6H) ; 1,20(d,3H). |
| 21 sel | DMSOd6 : 9,03(s,1H) ; 8,90(m,1H) ; 8,81(m,1H) ; 8,63(m,1H) ; 8,42(m,1H) ; 8,10(m,1H) ; 7,85(m,2H) ; 6,84(s,2H) ; 6,77(s,1H) ; 5,29(s,2H) ; 5,12(s,2H) ; 2,20(s,3H) ; 1,91(s,6H). |
| 22 sel | DMSOd6 : 9(m,4H) ; 8,6(m,2H) ; 8,3(m,2H) ; 6,9(s,2H) ; 6,8(s,1H) ; 5,2(s,4H) ; 2,3(s,3H) ; 2,06(s,6H). |

TABLEAU 6 (suite 3)

| Exemple N° (amine ou sel) | δ(ppm) |
|---|---|
| 23 sel | DMSOd6 : 9,30(s,1H) ; 8,67(m,2H) ; 8,47(m,1H) ; 8,04(m,1H) ; 7,87(s,1H) ; 7,70(s,1H) ; 6,85(s,2H) ; 6,71(s,1H) ; 5,01(s,2H) ; 4,35(t,2H) ; 3,61(t,2H) ; 2,28(m,2H) ; 2,24(s,3H) ; 1,99(s,6H). |
| 24 sel | DMSOd6 : 8,55(s,1H) ; 8,46(m,1H) ; 7,72(m,1H) ; 7,35(m,1H) ; 6,65(s,2H) ; 6,63(s,3H) ; 6,5(s,1H) ; 4,73(s,2H) ; 3,62(m,2H) ; 3,51(m,4H) ; 2,59(m,2H) ; 2,43(m,4H) ; 2,22(s,3H) ; 2,02(s,6H). |
| 25 sel | DMSOd6 : 8,55(s,1H) ; 8,46(m,1H) ; 7,72(m,1H) ; 7,37(m,1H) ; 6,6(s,2H) ; 6,37(s,1H) ; 4,7(s,2H) ; 3,6(m,2H) ; 2,65(m,2H) ; 2,3(s,6H) ; 2,2(s,3H) ; 2,15(s,6H) ; 1,92(s,6H). |
| 26 amine | DMSOd6 : 8,54(m,2H) ; 7,7(m,1H) ; 7,45(m,1H) ; 6,85(s,2H) ; 6,49(s,1H) ; 4,74(s,2H) ; 3,49(t,2H) ; 2,6(m,2H) ; 2,43(q,4H) ; 2,22(s,3H) ; 2,02(s,6H) ; 0,9(t,6H). |
| 27 sel | DMSOd6 : 9(s,1H) ; 8,88(m,1H) ; 8,67(m,1H) ; 8,09(m,1H) ; 6,88(s,2H) ; 6,78(s,1H) ; 5,55(q,1H) ; 3,64(t,2H) ; 3,09(t,2H) ; 2,70(s,6H) ; 2,23(s,3H) ; 2,10(m,2H) ; 2,02(s,6H) ; 1,83(d,3H). |
| 28 amine | DMSOd6 : 8,52(s,1H) ; 8,48(m,1H) ; 7,70(m,1H) ; 7,58(s,1H) ; 7,36(m,1H) ; 7,11(s,1H) ; 6,87(s,3H) ; 6,52(s,1H) ; 5,40(q,1H) ; 3,98(t,2H) ; 3,31(t,2H) ; 2,23(s,3H) ; 2,02(s,6H) ; 1,95(m,2H) ; 1,62(d,3H). |

TABLEAU 6 (suite 4)

| Exemple N° (amine ou sel) | δ(ppm) |
|---|---|
| 29 sel | DMSOd6 : 8,53(m,1H) ; 8,48(m,1H) ; 7,70(m,1H) ; 7,36(m,1H) ; 6,85(s,2H) ; 6,61(s,3H) ; 6,50(s,1H) ; 4,72(s,2H) ; 3,55(m,4H) ; 3,50(t,2H) ; 2,37(m,6H) ; 2,22(s,3H) ; 2,02(s,6H) ; 1,79(m,2H). |
| 30 sel | DMSOd6 : 8,9(m,2H) ; 8,64(m,1H) ; 8,09(m,1H) ; 6,86(s,2H) ; 6,73(s,1H) ; 5,46(q,1H) ; 3,99(t,2H) ; 3,35(m,4H) ; 3,12(m,2H) ; 2,21(s,3H) ; 2(s,6H) ; 1,83(d,3H). |
| 31 sel | DMSOd6 : 9,00(s,1H) ; 8,89(d,1H) ; 8,62(d,1H) ; 8,1(m,1H) ; 7,5-7,3(m,5H) ; 4,98(s,2H) ; 4(t,2H) ; 3,43(m,2H) ; 2,81(s,6H) ; 2,74(t,2H) ; 1,51(m,2H) ; 1,25(m,6H) ; 0,82(t,3H). |
| 32 amine | CDCl3 : 8,70(m,2H) ; 8,55(m,2H) ; 7,8(m,2H) ; 7,30(m,2H) ; 6,9(s,2H) ; 6,25(s,1H) ; 5,35(q,2H) ; 2,35(s,3H) ; 2,1(s,6H) ; 1,6(d,6H). |
| 33 sel | DMSOd6 : 8,55(s,1H) ; 8,5(m,1H) ; 7,8(m,1H) ; 7,35(m,1H) ; 6,85(s,2H) ; 6,61(s,3H) ; 6,5(s,1H) ; 5,31(m,1H) ; 3,5(m,6H) ; 2,52(m,1H) ; 2,4(m,5H) ; 2,21(s,3H) ; 2,05(s,6H) ; 1,65(m,3H). |
| 34 sel | DMSOd6 : 8,85(m,2H) ; 8,50(m,1H) ; 8,01(m,1H) ; 6,86(s,2H) ; 6,72(s,1H) ; 5,35(m,1H) ; 4,01(m,2H) ; 3,88(m,1H) ; 3,47(m,2H) ; 3,04(m,2H) ; 2,22(s,3H) ; 2,15-1,7(m,13H) ; 1,15(m,3H). |

TABLEAU 6 (suite 5)

| Exemple N° (amine ou sel) | δ(ppm) |
|---|---|
| 35 sel | DMSOd6 : 8,9(m,2H) ; 8,6(m,1H) ; 8,07(m,1H) ; 6,87(s,2H) ; 6,72(s,1H) ; 5,48(q,1H) ; 4(m,2H) ; 3,45(m,2H) ; 3,28(m,2H) ; 2,92(m,2H) ; 2,23(s,3H) ; 2,00(s,6H) ; 1,81(d,3H) ; 1,70(m,6H). |
| 36 sel | DMSOd6 : 8,96(s,1H) ; 8,89(m,1H) ; 8,57(m,1H) ; 8,10(m,1H) ; 6,87(s,2H) ; 6,75(s,1H) ; 5,09(s,2H) ; 4,12(m,2H) ; 3,61(m,2H) ; 3,42(m,2H) ; 2,22(s,3H) ; 2,02(s,6H) ; 1,33(m,12H). |
| 37 sel | DMSOd6 : 8,95(s,1H) ; 8,89(m,1H) ; 8,62(m,1H) ; 8,10(m,1H) ; 6,87(s,2H) ; 6,72(s,1H) ; 5,39(m,1H) ; 4,04(m,2H) ; 3,60(m,2H) ; 3,30(m,2H) ; 2,23(s,3H) ; 2,02(s,6H) ; 1,82(m,3H) ; 1,33(m,3H) ; 1,26(m,9H). |
| 38 sel | DMSOd6 : 8,64(s,1H) ; 8,48(m,1H) ; 7,8(m,1H) ; 7,66(s,2H) ; 7,35(m,1H) ; 7,31(s,1H) ; 7,17(s,1H) ; 6,59(s,2H) ; 4,73(s,2H) ; 3,75(m,2H) ; 2,75(m,2H) ; 2,38(s,6H) ; 1,3(s,18H). |
| 39 amine | CDCl3 : 8,62(m,1H) ; 8,53(m,1H) ; 7,75(m,1H) ; 7,65(s,2H) ; 7,25(m,1H) ; 6,54(s,1H) ; 5,24(s,1H) ; 4,79(s,2H) ; 3,58(t,2H) ; 2,59(t,2H) ; 2,28(s,6H) ; 1,46(s,18H). |
| 40 sel | DMSOd6 : 9,00(s,1H) ; 8,95(m,1H) ; 8,64(m,1H) ; 8,13(m,1H) ; 6,87(s,2H) ; 6,74(s,1H) ; 5,35(m,1H) ; 3,94(m,2H) ; 3,32(m,2H) ; 3,08(m,4H) ; 2,22(s,3H) ; 2,02(s,6H) ; 1,81(m,3H) ; 1,54(m,4H) ; 1,21(m,4H) ; 0,8(t,6H). |

TABLEAU 6 (suite 6)

| Exemple N° (amine ou sel) | δ(ppm) |
|---|---|
| 41 amine | DMSOd6 : 8,56(s,1H) ; 8,46(m,1H) ; 7,74(m,1H) ; 7,35(m,1H) ; 6,99(s,2H) ; 6,50(s,1H) ; 5,40(m,1H) ; 3,41(m,2H) ; 2,88(m,1H) ; 2,69(m,2H) ; 2,5-2,3(m,2H) ; 2,10(s,6H) ; 1,65(d,3H) ; 1,21(m,6H) ; 1,05(m,12H). |
| 42 amine | DMSOd6 : 8,43(m,2H) ; 7,64(m,1H) ; 7,32(m,1H) ; 7,02(s,2H) ; 6,49(s,1H) ; 3,64(t,2H) ; 3,49(t,2H) ; 2,99(m,2H) ; 2,88(m,1H) ; 2,73(m,2H) ; 2,50(m,2H) ; 2,22(s,6H) ; 1,22(d,6H) ; 1,09(d,12H). |
| 43 sel | DMSOd6 : 8,50(m,2H) ; 7,70(m,1H) ; 7,35(m,1H) ; 7(s,2H) ; 6,55(s,1H) ; 4,75(s,2H) ; 3,5(t,2H) ; 3,05(t,2H) ; 2,95(m,3H) ; 2,75(s,6H) ; 2,05(m,2H) ; 1,2(d,6H) ; 1,05(d,12H). |
| 44 amine | DMSOd6 : 8,55(s,1H) ; 8,45(m,1H) ; 7,73(m,1H) ; 7,34(m,1H) ; 7,00(s,2H) ; 6,54(s,1H) ; 4,72(s,2H) ; 3,6(m,2H) ; 3,35(m,2H) ; 2,77(m,1H) ; 2,7(m,2H) ; 2,5(m,4H) ; 1,65(m,4H) ; 1,20(d,6H) ; 1,06(d,12H). |
| 45 sel | DMSOd6 : 8,55(m,2H) ; 7,71(m,1H) ; 7,40(m,1H) ; 7,01(s,2H) ; 6,65(s,1H) ; 4,74(s,2H) ; 3,68(m,2H) ; 3,30(m,2H) ; 3,15(m,4H) ; 2,87(m,1H) ; 2,68(m,2H) ; 1,65(m,4H) ; 1,47(m,2H) ; 1,20(d,6H) ; 1,08(d,12H). |
| 46 sel | DMSOd6 : 8,44(m,2H) ; 7,70(m,1H) ; 7,30(m,1H) ; 7,15(m,2H) ; 7,01(s,2H) ; 6,70(m,2H) ; 6,45(s,1H) ; 5,06(s,2H) ; 2,91(s,6H) ; 2,8(m,3H) ; 1,20(d,6H) ; 1,07(d,12H). |

TABLEAU 6 (suite 7)

| Exemple N° (amine ou sel) | δ(ppm) |
|---|---|
| 47 sel | DMSOd6 : 8,55(m,2H) ; 7,65(m,1H) ; 7,26(m,1H) ; 7,02(s,2H) ; 6,25(s,1H) ; 4,75(s,2H) ; 3,39(t,2H) ; 2,82(m,3H) ; 2,33(t,2H) ; 2,25(s,6H) ; 1,70(m,2H) ; 1,51(m,2H) ; 1,24(d,6H) ; 1,16(d,12H). |
| 48 sel | DMSOd6 : 8,52(m,2H) ; 7,71(m,1H) ; 7,40(m,1H) ; 6,35(s,1H) ; 4,85(s,2H) ; 3,86(t,2H) ; 3,34(t,2H) ; 2,84(s,6H) ; 1,92-1,37(m,1H). |
| 49 sel | DMSOd6 : 8,63(s,1H) ; 8,5(m,1H) ; 7,8(m,1H) ; 7,4(m,1H) ; 7,3(s,1H) ; 7,1(s,2H) ; 4,73(s,2H) ; 4(m,2H) ; 3,83(s,6H) ; 3,68(s,3H) ; 3,43(m,2H) ; 2,89(s,6H). |
| 50 sel | DMSOd6 : 8,58(s,1H) ; 8,5(m,1H) ; 7,7(m,2H) ; 7,5(m,3H) ; 4,68(s,2H) ; 3,8(t,2H) ; 3,26(t,2H) ; 2,73(s,6H) ; 2,1(s,3H). |
| 51 sel | DMSOd6 : 8,5(m,2H) ; 7,7(d,1H) ; 7,4(m,1H) ; 7(m,3H) ; 4,7(s,2H) ; 3,84(m,2H) ; 3,35(m,2H) ; 2,8(s,6H) ; 2,5(s,3H) ; 2,3(s,3H) ; 1,1(d,6H). |
| 52 base | DMSOd6 : 8,61(m,1H) ; 8,48(m,1H) ; 7,76(m,3H) ; 7,36(m,1H) ; 7,20(m,2H) ; 7,07(s,1H) ; 4,77(s,2H) ; 3,60(t,2H) ; 2,53(t,2H) ; 2,15(s,6H) ; 1,78(m,6H). |
| 53 sel | DMSOd6 : 8,35(m,1H) ; 7,6(m,1H) ; 7,42(m,1H) ; 6,86(s,2H) ; 6,62(s,1H) ; 4,74(s,2H) ; 3,9(m,2H) ; 3,3(m,2H) ; 2,73(s,6H) ; 2,23(s,3H) ; 2,01(s,6H). |

TABLEAU 6 (suite 8)

| Exemple N° (amine ou sel) | δ(ppm) |
|---|---|
| 54 sel | DMSOd6 : 8,57(m,2H) ; 7,73(m,1H) ; 7,40(m,1H) ; 6,99(s,1H) ; 6,85(s,1H) ; 4,73(s,2H) ; 3,91(t,2H) ; 3,31(t,2H) ; 2,77(s,6H) ; 2,50(s,3H) ; 2,35(s,3H). |
| 55 sel | DMSOd6 : 8,55(m,2H) ; 7,70(m,1H) ; 7,35(m,1H) ; 7,15(s,2H) ; 6,60(s,1H) ; 4,70(s,2H) ; 3,87(t,2H) ; 3,30(t,2H) ; 2,75(s,6H) ; 2,02(s,6H) ; 2,01(s,3H). |
| 56 sel | DMSOd6 : 8,53(m,2H) ; 7,72(m,1H) ; 7,57(s,2H) ; 7,40(m,1H) ; 6,80(s,1H) ; 4,75(s,2H) ; 3,88(t,2H) ; 3,30(t,2H) ; 2,75(s,6H) ; 2,13(s,6H). |
| 57 sel | DMSOd6 : 8,53(m,2H) ; 7,74(m,1H) ; 7,59(s,2H) ; 7,44(m,1H) ; 6,72(s,1H) ; 4,75(s,2H) ; 3,85(m,2H) ; 3,8(m,0,5H) ; 3,37(m,2H) ; 2,80(s,6H) ; 2,13(s,6H) ; 1,05(d,3H). |
| 58 sel | DMSOd6 : 8,53(m,2H) ; 7,72(m,1H) ; 7,41(m,1H) ; 7,12(m,3H) ; 6,66(s,1H) ; 4,74(s,2H) ; 3,90(t,2H) ; 3,36(t,2H) ; 2,80(s,6H) ; 2,09(s,6H). |
| 59 sel | DMSOd6 : 8,55(m,2H) ; 7,72(m,1H) ; 7,27(m,6H) ; 6,40(s,1H) ; 4,84(s,2H) ; 3,83(m,4H) ; 2,75(s,2H) ; 2,57(s,6H). |
| 60 sel | DMSOd6 : 8,86(m,2H) ; 8,50(m,1H) ; 8,04(m,1H) ; 7,20(s,2H) ; 4,87(s,2H) ; 3,95(m,2H) ; 3,42(m,2H) ; 3,25(m,1H) ; 2,87(s,6H) ; 1,36(s,18H) ; 1,23(d,6H). |

TABLEAU 6 (suite 9)

| Exemple N° (amine ou sel) | δ(ppm) |
|---|---|
| 61 sel | DMSOd6 : 8,55(m,2H) ; 7,74(m,1H) ; 7,68(s,2H) ; 7,40(m,1H) ; 6,76(s,1H) ; 4,75(s,2H) ; 3,86(m,5H) ; 3,40(m,2H) ; 2,80(s,6H) ; 2,16(s,6H). |
| 62 sel | DMSOd6 : 8,63-7,12(m,9H) ; 6,95(s,1H) ; 4,75(s,2H) ; 3,99(t,2H) ; 3,82(s,3H) ; 3,43(t,2H) ; 2,87(d,6H). |
| 63 sel | DMSOd6 : 8,64-7,49(m,11H) ; 7,44(s,1H) ; 4,78(s,2H) ; 4,05(t,2H) ; 3,47(t,2H) ; 2,91(s,6H). |
| 69 sel | DMSOd6 : 8,66(s,1H) ; 8,63(m,1H) ; 7,97(m,1H) ; 7,63(m,1H) ; 6,67(s,2H) ; 6,65(s,1H) ; 4,8(s,2H) ; 3,86(m,2H) ; 3,59(m,2H) ; 3,48(m,2H) ; 3,07(m,2H) ; 2,23(s,3H) ; 2,03(s,6H) ; 1,95(m,2H) ; 1,81(m,2H). |
| 70 sel | DMSOd6 : 8,54(m,1H) ; 8,47(m,1H) ; 7,72(m,1H) ; 7,38(m,1H) ; 6,66(s,2H) ; 6,53(s,1H) ; 4,74(s,2H) ; 3,61(t,2H) ; 3,07(m,4H) ; 2,68(m,9H) ; 2,23(s,3H) ; 2,02(s,6H). |
| 71 amine | CDCl3 : 8,90(m,1H) ; 8,10(m,2H) ; 7,65-7,8(m,2H) ; 7,55(m,1H) ; 6,89(s,2H) ; 6,27(s,1H) ; 4,99(s,2H) ; 3,59(t,2H) ; 2,61(t,2H) ; 2,28(s,3H) ; 2,26(s,6H) ; 2,17(s,6H). |

TABLEAU 6 (suite 10)

| Exemple N° (amine ou sel) | δ(ppm) |
|---|---|
| 72 sel | DMSOd6 : 7,9(m,1H) ; 7,5(m,1H) ; 7,4(m,2H) ; 7,0(s,2H) ; 6,55(s,1H) ; 4,8(s,2H) ; 4,1(t,2H) ; 3,45(t,2H) ; 2,66(s,6H) ; 2,65(m,3H) ; 1,20(d,6H) ; 1,05(d,12H). |
| 73 amine | DMSOd6 : 7,56(d,1H) ; 7,42(d,1H) ; 7,38(s,2H) ; 7,18(t,1H) ; 7,01(t,1H) ; 6,89(s,2H) ; 6,47(s,1H) ; 4,80(s,2H) ; 3,77(s,3H) ; 3,40(m,2H) ; 2,43(m,2H) ; 2,25(s,3H) ; 2,13(s,12H). |
| 74 sel | DMSOd6 : 8,85(m,2H) ; 8,45(m,1H) ; 8,02(m,1H) ; 7,01(s,2H) ; 6,72(s,1H) ; 4,99(s,2H) ; 3,78(t,2H) ; 2,87(m,3H) ; 2,60(m,4H) ; 1,15(m,21H). |
| 75 sel | DMSOd6 : 8,55(m,2H) ; 7,70(m,2H) ; 7,37(m,2H) ; 6,85(s,2H) ; 6,50(s,1H) ; 4,67(s,2H) ; 4,40(s,2H) ; 3,7(m,1H) ; 2,95(s,3H) ; 2,83(s,3H) ; 2,22(s,3H) ; 2,01(s,6H) ; 1,03(d,6H). |
| 76 base | DMSOd6 : 8,61(d,1H) ; 8,50(m,1H) ; 7,80(d,1H) ; 7,38(m,1H) ; 7,0(s,2H) ; 6,58(s,1H) ; 4,73(s,2H) ; 4,36(s,2H) ; 3,61(s,3H) ; 2,85(m,1H) ; 2,63(m,2H) ; 1,20(d,6H) ; 1,05(d,12H). |
| 77 base | DMSOd6 : 8,48(m,2H) ; 7,65(m,1H) ; 7,34(m,1H) ; 7,0(s,2H) ; 6,53(s,1H) ; 4,75(s,2H) ; 3,59(m,4H) ; 3,23(s,3H) ; 2,80(m,3H) ; 1,20(d,6H) ; 1,06(d,12H). |

TABLEAU 6 (suite 11)

| Exemple N° (amine ou sel) | δ(ppm) |
|---|---|
| 83 sel | DMSOd6 : 8,55(m,2H) ; 7,75(m,1H) ; 7,43(m,1H) ; 6,90(s,2H) ; 4,78(s,2H) ; 3,94(t,2H) ; 3,35(t,2H) ; 2,77(s,6H) ; 2,26(s,3H) ; 2,09(s,3H) ; 2,03(s,6H). |
| 84 sel | DMSOd6 : 8,63(s,1H) ; 8,54(m,1H) ; 8,12(m,2H) ; 7,80(m,1H) ; 7,40(m,4H) ; 4,78(s,2H) ; 4,05(t,2H) ; 3,42(t,2H) ; 2,85(s,6H). |
| 85 sel | DMSOd6 : 8,8(m,2H) ; 8,37(m,1H) ; 7,97(m,1H) ; 6,85(s,2H) ; 4,96(s,2H) ; 3,7(s,3H) ; 3,6(m,4H) ; 2,2(s,3H) ; 2(s,6H). |

## Revendications

**1.** Composés de formule

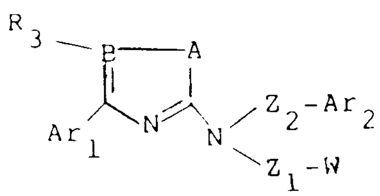

I

dans laquelle
A représente O ou S ;
B représente C ou N ;
$Z_1$ représente alkylène en $C_1$ à $C_4$ ou phénylène ;
$Z_2$ représente alkylène en $C_1$ à $C_4$ ;
W représente $NR_1R_2$, dans lequel $R_1$ représente H, alkyle en $C_1$ à $C_4$ et $R_2$ représente H, alkyle en $C_1$ à $C_4$, $CONQ_1Q_2$ ou $CSNQ_1Q_2$ dans lesquels $Q_1$ et $Q_2$ représentent indépendamment H ou alkyle en $C_1$ à $C_4$, $SO_2Q_3$ ou $COQ_3$ dans lesquels $Q_3$ représente alkyle en $C_1$ à $C_4$, $COOQ_4$ dans lequel $Q_4$ représente alkyle en $C_1$ à $C_4$ ou benzyle, ou $R_1$ et $R_2$ considérés ensemble forment avec N un hétérocycle saturé morpholine, pyrrolidine, pipéridine, pipérazine ou ($C_1$-$C_3$)alkyl-4 pipérazine, ou W représente le N-oxyde des amines $NR_1R_2$, ou encore W représente alkoxy ou thioalkoxy en $C_1$ à $C_4$, $CONQ_1Q_2$, $CSNQ_1Q_2$, pyridyle, imidazolyle ou $COOQ_5$ dans lesquel $Q_5$ représente alkyle en $C_1$ à $C_5$,
$R_3$ n'est pas présent lorsque B est N, ou représente H, alkyle en $C_1$ à $C_8$ ou halogéno lorsque B est C;
$Ar_1$ représente un phényle éventuellement substitué par un ou des groupes choisis parmi halogéno, alkyle, alkoxy ou thioalkoxy en $C_1$ à $C_4$, hydroxy, carboxy, $COOQ_6$ ou $COSQ_6$ ou $CSOQ_6$ dans lesquels

$Q_6$ est un alkyle en $C_1$ à $C_4$, carboxamido, cyano, amino acétamido, nitro, trifluorométhyle, ou $Ar_1$ représente un hétérocycle aromatique thiényle, furyle, indolyle ou encore $Ar_1$ représente naphtyle, benzyle ou cyclohexyle, ou
$Ar_1$ et
$R_3$ considérés ensemble forment un groupe

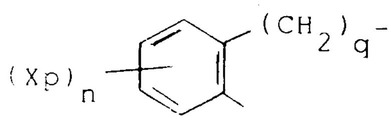

tel que le carbone du phényle soit lié en position 4 de l'hétérocycle et dans lequel q vaut 2 à 4, Xp identiques ou différents représentent H, alkyle en $C_1$ à $C_3$ ou halogèno et n représente 1 à 3 ;
$Ar_2$ représente un hétérocycle aromatique azote, pyrimidinyle, quinolyle, isoquinolyle, indolyle, isoindolyle ou pyridyle, éventuellement substitués par alkyle ou alkoxy en $C_1$ à $C_3$ ou halogéno ;
les sels de ces composés avec des acides ou des bases, et lorsque A, B, $R_3$, $Ar_1$, $Ar_2$, $Z_1$ et $Z_2$ ont les significations suivantes :
A est S,
B est C,
$Z_1$ est défini comme ci-dessus,
$Z_2$ représente un groupe alkylène en $C_1$-$C_3$,
$R_3$ représente un atome d'hydrogène ou un groupe alkyle en $C_1$-$C_8$,
$Ar_1$ représente un groupe phényle portant éventuellement un ou plusieurs substituants choisis parmi les atomes d'halogène, les groupes alkyle ou alkoxy en $C_1$ à $C_4$, les groupes nitro, trifluorométhyle, ou hydroxy, ou $Ar_1$ et $R_3$ considérés ensemble forment le groupe

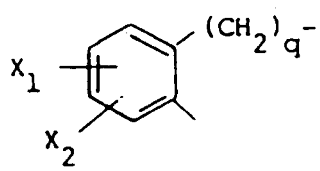

dans lequel q vaut 2 à 4, et $X_1$ et $X_2$ indépendamment l'un de l'autre représentent l'atome d'hydrogène, un halogène, ou un groupe méthyle, et
$Ar_2$ représente un groupe pyrimidinyle, quinolyle, isoquinolyle, indolyle ou pyridyle éventuellement substitués par un groupe alkyle ou alkoxy en $C_1$ à $C_3$ ou un atome d'halogène,
et W représente un groupe $NR_1R_2$ dans laquelle $R_1$ et $R_2$, indépendamment l'un de l'autre, représentent l'atome d'hydrogène, un groupe alkyle en $C_1$ à $C_4$ ou un phényle ou $R_1$ et $R_2$ considérés ensemble forment avec l'atome d'azote auxquels ils sont rattachés un hétérocycle saturé tel que morpholine, pyrrolidine, pipéridine, pipérazine ou ($C_1$-$C_3$)alkyl-4 pipérazine, ou W représente un groupe pyridyle ou imidazolyle, ainsi que les sels d'addition de ces derniers composés avec des acides pharmaceutiquement acceptables.

**2.** Composé selon la revendication 1, de formule I dans laquelle B représente l'atome de carbone, et ses sels.

**3.** Composé selon la revendication 1, de formule I dans laquelle B représente l'atome de carbone et A celui de soufre, et ses sels.

**4.** Composé selon la revendication 1, de formule I dans laquelle B représente C, A représente S, $Ar_1$ représente un phényle au moins orthosubstitué, et ses sels.

**5.** Composé selon la revendication 1, de formule I dans laquelle B représente C, A représente S, $Ar_1$ représente un phényle sans substituant en ortho et $R_3$ représente alkyle en $C_1$ à $C_3$ et ses sels.

**6.** Composés selon la revendication 1 dans lesquels $Ar_2$ représente un groupe pyridyle.

**7.** Composés selon la revendication 1 dans lesquels $Ar_2$ représente un groupe pyridyl-2 ou -3, $Z_1$ représente un groupe alkylène en $C_2$ à $C_4$ et W représente le groupe $NR_1R_2$

**8.** Composé selon l'une dos revendications 1 à 5, de formule I dans laquelle W représente alkoxy ou thioalkoxy en $C_1$-$C_2$, et ses sels.

**9.** Composé selon l'une des revendications 1 à 5 de formule I dans laquelle W représente un groupe amino et ses sels.

**10.** Composé selon l'une des revendications 1 à 7 de formule I dans laquelle $Z_1$ est un alkylène en $C_2$ ou $C_3$ et ses sels.

**11.** Composé selon la revendication 1 choisi parmi
- N-[N',N'-diméthylamino-2 éthyl] N-[pyridyl-3 méthyl] amino-2 (triisopropyl-2,4,6 phényl)-4 thiazole,
- N-[N',N'-diméthylamino-2 éthyl] N-[pyridyl-3 méthyl] amino-2 (triméthyl-2,4,6 phényl)-4 thiazole,
- N-[N',N'-diméthylamino-2 éthyl] N-[pyridyl-3 méthyl] amino-2 (dichloro-2,4 phényl)-4 méthyl-5 thiazole
- N-[N',N'-diméthylamino-2 éthyl] N-[pyridyl-3 méthyl] amino-2 (dichloro-2,4 phényl)-4 méthyl-5 oxazole
- N-[N',N'-diméthylamino-2 éthyl] N-[pyridyl-3 méthyl] amino-2 (triisopropyl-2,4,6 phényl)-4 chloro-5 thiazole
- N-[N',N'-diméthylamino-2 éthyl] N-[pyridyl-3 méthyl] amino-2 (triisopropyl-2,4,6 phényl)-4 bromo-5 thiazole et leurs sels.

**12.** Procédé de préparation des composés de formule I des revendications 1 à 11, caractérisé en ce qu'on effectue une réaction de substitution entre les amines primaires $H_2NZ_1W$ ou $H_2N$-$Z_2$ $Ar_2$ ou l'amine secondaire de formule IV

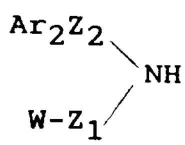

et l'hétérocycle de formule V

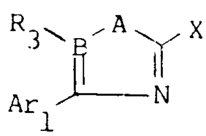

dans lequel X représente un groupe halogéno ou sulfonate, réaction suivie lorsque l'amine es primaire, de la substitution de l'amine secondaire obtenue par action, selon le cas, de W-$Z_1$-Y ou $Ar_2$-$Z_2$-Y dans lesquels Y représente halogéno ou sulfonate avec pour A,B,$R_3$,$Ar_1$,$Z_2$,$Ar_2$,$Z_1$,W les mêmes significations que dans la formule I, à l'exclusion de $R_3$ = halogéno, ou $Ar_1$,$Ar_2$,W représentent des groupes correspondants à ceux de la formule I dans lesquels les fonctions réactives ont été protégées, auquel cas elles sont déprotégées après la substitution.

**13.** Procédé de préparation des composés de formule I des revendications 1 à 11, dans laquelle A représente S et B représente C, caractérisé en ce que l'on condense une thiourée de formule VI

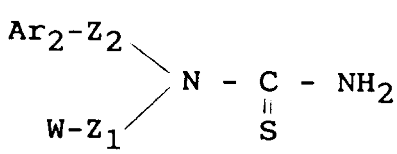

avec une alpha halogénocétone de formule VII

$Ar_1$ - CO - CHX - $R_3$

dans lesquelles $Ar_1$,$Ar_2$, $Z_1$,$Z_2$,$R_3$ et W ont les mêmes significations que dans la formule I, à l'exclusion de $R_3$ = halogéno, ou représentent des groupes correspondants dans lesquels les fonctions réactives ont été protégés, auquel cas elles sont déprotégées après la condensation.

**14.** Procédé de préparation des composés de formule I des revendications 1 à 11, dans laquelle A représente S et B représente C, caractérisé en ce que l'on condense une amine de formule IV

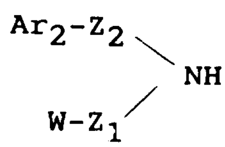

avec une alpha thiocyanatocétone de formule VIII

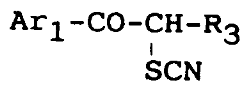

dans lesquelles $Ar_1$, $Ar_2$, $Z_1$, $Z_2$, $R_3$ et W ont les mêmes significations que dans la formule I, à l'exclusion de $R_3$ = halogéno, ou représentent des groupes correspondants dans lesquels les fonctions réactives ont été protégées, auquel cas elles sont déprotégées après la condensation.

**15.** Procédé de préparation des composés de formule I des revendications 1 à 11, dans laquelle W représente $NR_1R_2$ avec $R_2$ qui représente $CONQ_1Q_2$, $CSNQ_1Q_2$, $SO_2Q_3$, $COQ_3$, $Q_1$, $Q_2$ et $Q_3$ ayant la même signification qu'à la revendication 1, caractérisé en ce que l'on fait réagir sur le composé correspondant dans lequel $R_2$ représente H, obtenu par l'un des procédés selon les revendications 12 à 14, un dérivé réactif de $R_2$.

**16.** Procédé de préparation des composés de formule I des revendications 1 à 11, dans laquelle A représente S ou O, B représente C, $R_3$ représente halogéno, caractérisé en ce que l'on effectue l'halogénation du composé de formule I correspondant dans lequel $R_3$ est H, obtenu par l'un des procédés selon les revendications 12 à 15, par action de l'halogène.

**17.** Composition pharmaceutique caractérisée en ce qu'elle comporte au moins un composé selon l'une des revendications 1 à 11, dans lesquelles les sels sont pharmaceutiquement acceptables, et au moins un excipient.

**18.** Composition pharmaceutique selon la revendication 17 pour le traitement des pathologies associées à un excès de PAF-acéther comportant une quantité efficace d'un composé selon l'une des revendications 1 à 11.

## Claims

**1.** Compounds of formula:

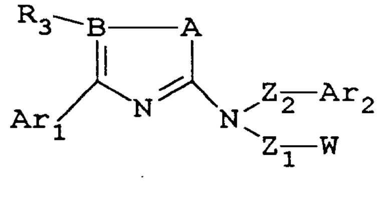

I

in which
A represents O or S;
B represents C or N;
$Z_1$ represents $C_1$-$C_4$ alkylene or phenylene;
$Z_2$ represents $C_1$-$C_4$ alkylene;
W represents $NR_1R_2$, in which $R_1$ represents H or $C_1$-$C_4$ alkyl and $R_2$ represents H, $C_1$-$C_4$ alkyl, $CONQ_1Q_2$ or $CSNQ_1Q_2$ in which $Q_1$ and $Q_2$ independently represent H or $C_1$-$C_4$ alkyl, $SO_2Q_3$ or $COQ_3$ in which $Q_3$ represents $C_1$-$C_4$ alkyl, $COOQ_4$ in which $Q_4$ represents $C_1$-$C_4$ alkyl or benzyl, or $R_1$ and $R_2$ taken together form with N a saturated heterocycle morpholine, pyrrolidine, piperidine, piperazine or 4-($C_1$-$C_3$)alkyl-piperazine, or W represents the N-oxide of the amines $NR_1R_2$, or W is also selected from $C_1$-$C_4$ alkoxy or thioalkoxy, $CONQ_1Q_2$ or $CNSQ_1Q_2$, pyridyl, imidazolyl or $COOQ_5$ in which $Q_5$ is $C_1$-$C_5$ alkyl;
$R_3$ is not present when B is N, or represents H, $C_1$-$C_8$ alkyl or halogen when B is C;
$Ar_1$ represents phenyl optionally substituted by one or more groups selected from halogen, $C_1$-$C_4$ alkyl, $C_1$-$C_4$ alkoxy, $C_1$-$C_4$ thioalkoxy, hydroxy, carboxy, $COOQ_6$ or $COSQ_6$ or $CSOQ_6$ in which $Q_6$ is $C_1$-$C_4$ alkyl, carboxamido, cyano, amino, acetamido, nitro or trifluoromethyl, or $Ar_1$ represents an aromatic heterocycle thienyl, furyl or indolyl, or $Ar_1$ may also represent naphthyl, benzyl or cyclohexyl, or $Ar_1$ and $R_3$ taken together form a group

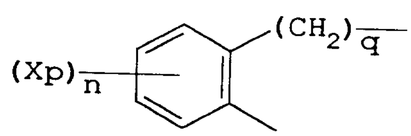

such that the carbon of the phenyl is linked to position 4 of the heterocycle and in which q is equal to 2 to 4, Xp, identical or different, are selected from H, $C_1$-$C_3$ alkyl or halogen and n is 1 to 3;
$Ar_2$ represents a nitrogenous aromatic heterocycle, pyrimidinyl, quinolyl, isoquinolyl, indolyl, isoindolyl or pyridyl, optionally substituted by $C_1$-$C_3$ alkyl or alkoxy or halogen;
as well as the salts of these compounds with acids or bases;
and when A, B, $R_3$, $Ar_1$, $Ar_2$, $Z_1$ and $Z_2$ have the following meanings:
A is S,
B is C,
$Z_1$ is defined as above,
$Z_2$ represents a $C_1$-$C_3$ alkylene group,
$R_3$ represents a hydrogen atom or a $C_1$-$C_8$ alkyl group,
$Ar_1$ represents a phenyl group optionally bearing one or more substituents selected from halogen atoms, $C_1$-$C_4$ alkyl or alkoxy groups and nitro, trifluoromethyl or hydroxy groups;
or $Ar_1$ and $R_3$ taken together form the group

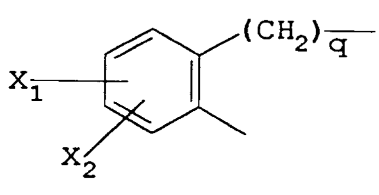

in which q is equal to 2 to 4, and $X_1$ and $X_2$ each independently represent a hydrogen atom, a halogen or a methyl group, and
$Ar_2$ represents a pyrimidinyl, quinolyl, isoquinolyl, indolyl or pyridyl group optionally substituted by a $C_1$-$C_3$ alkyl or alkoxy group or a halogen atom, and W represents a $NR_1R_2$ group in which $R_1$ and $R_2$ each independently represents a hydrogen atom, a $C_1$-$C_4$ alkyl or phenyl group, or $R_1$ and $R_2$ taken together form with the nitrogen atom to which they are attached a saturated heterocycle such as morpholine, pyrrolidine, piperidine, piperazine or 4-($C_1$-$C_3$)alkyl piperazine, or
W represents a pyridyl or imidazolyl group, as well as the addition salts of these latter compounds with

pharmaceutically acceptable acids.

2. A compound of formula I according to claim 1, in which B represents a carbon atom, and the salts thereof.

3. A compound of formula I according to claim 1, in which B represents a carbon atom and A a sulphur atom, and the salts thereof

4. A compound of formula I according to claim 1, in which B represents C, A represents S, $Ar_1$ represents an at least ortho-substituted phenyl, and the salts thereof.

5. A compound of formula I according to claim 1, in which B represents C, A represents S, $Ar_1$ represents phenyl without an ortho substituent and $R_3$ represents $C_1$-$C_3$ alkyl, and the salts thereof.

6. Compounds according to claim 1 in which $Ar_2$ represents a pyridyl group.

7. Compounds according to claim 1 in which $Ar_2$ represents a 2-pyridyl or 3-pyridyl group, $Z_1$ represents a $C_2$-$C_4$ alkylene group and W represents the group $NR_1R_2$.

8. A compound of formula I according to one of claims 1 to 5, in which W represents $C_1$-$C_2$ alkoxy or thioalkoxy, and the salts thereof.

9. A compound of formula I according to one of claims 1 to 5, in which W represents an amino group, and the salts thereof.

10. A compound of formula I according to one of claims 1 to 7, in which $Z_1$ is $C_2$ or $C_3$ alkylene and the salts thereof.

11. A compound according to claim 1 selected from
- 2-(N-[2-N',N'-dimethylaminoethyl]-N-[3-pyridylmethyl]amino)-4-(2,4,6-triisopropylphenyl)thiazole,
- 2-(N-[2-N',N'-dimethylaminoethyl]-N-[3-pyridylmethyl]amino)-4-(2,4,6-trimethylphenyl)thiazole,
- 2-(N-[2-N',N'-dimethylaminoethyl]-N-[3-pyridylmethyl]amino)-4-(2,4-dichlorophenyl)-5-methyl thiazole
- 2-(N-[2-N',N'-dimethylaminoethyl]-N-[3-pyridylmethyl]amino)-4-(2,4-dichlorophenyl)-5-methyl oxazole
- 2-(N-[2-N',N'-dimethylaminoethyl]-N-[3-pyridylmethyl]amino)-4-(2,4,6-triisopropylphenyl)-5-chloro thiazole
- 2-(N-[2-N',N'-dimethylaminoethyl]-N-[3-pyridylmethyl]amino)-4-(2,4,6-triisopropylphenyl)-5-bromo thiazole,
and the salts thereof.

12. A process for the preparation of the compounds of formula I of claims 1 to 12, characterised in that a substitution reaction is carried out between the primary amines $H_2NZ_1W$ or $H_2N$-$Z_2$ $Ar_2$ or the secondary amine of formula IV

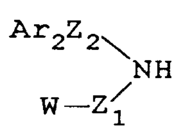

and the heterocycle of formula V

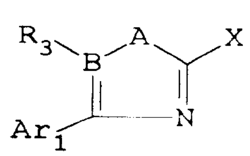

in which X represents a halogen or sulfonate group, a reaction followed, when the amine is primary, by

the substitution of the secondary amine obtained by reaction with either W-$Z_1$-Y or $Ar_2$-$Z_2$-Y in which Y represents halogen or sulphonate with A, B, $R_3$, $Ar_1$, $Z_2$, $Ar_2$, $Z_1$, W being as defined in formula I, with the exception of $R_3$ = halogen, or $Ar_1$, $Ar_2$, W represent equivalent groups to those of formula I in which the reactive functions have been protected, in which case they are deprotected after the substitution.

**13.** A process for the preparation of the compounds of formula I of claims 1 to 11, in which A represents S and B represents C, characterised in that a thiourea of formula VI

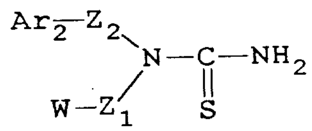

is condensed with an alpha halogenoketone of formula VII

$Ar_1$ - CO - CHX - $R_3$

in which $Ar_1$, $Ar_2$, $Z_1$, $Z_2$, $R_3$ and W are as defined in formula I, with the exception of $R_3$ = halogen, or represent equivalent groups in which the reactive functions have been protected, in which case they are deprotected after the condensation.

**14.** A process for the preparation of the compounds of formula I of claims 1 to 11, in which A represents S and B represents C, wherein an amine of formula IV

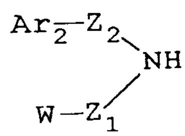

is condensed with an alpha thiocyanatoketone of formula VIII

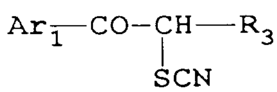

in which $Ar_1$, $Ar_2$, $Z_1$, $Z_2$, $R_3$ and W are as defined in formula I, with the exception of $R_3$ = halogen, or represent equivalent groups in which the reactive functions have been protected, in which case they are deprotected after the condensation.

**15.** A process for the preparation of the compounds of formula I of claims 1 to 11, in which W represents $NR_1R_2$ in which $R_2$ represents $CONQ_1Q_2$, $CSNQ_1Q_2$, $SO_2Q_3$ or $COQ_3$, $Q_1$, $Q_2$ and $Q_3$ being as defined in claim 1, characterised in that the corresponding compound in which $R_2$ is H, obtained by one of the processes according to claims 12 to 14, is reacted with a reactive derivative of $R_2$.

**16.** A process for the preparation of the compounds of formula I of claims 1 to 11, in which A represents S or O, B represents C, $R_3$ represents halogen, characterised in that the halogenation of the corresponding compound of formula I in which $R_3$ is H, obtained by one of the processes according to claims 12 to 15, is carried out by reaction with the halogen.

**17.** A pharmaceutical composition characterised in that it comprises at least one compound according to one of claims 1 to 11, in which the salts are pharmaceutically acceptable, and at least one excipient.

**18.** A pharmaceutical composition according to claim 17 for the treatment of diseases associated with an excess of PAF-acether containing an effective amount of a compound according to one of claims 1 to

11.

**Patentansprüche**

1. Verbindungen der Formel

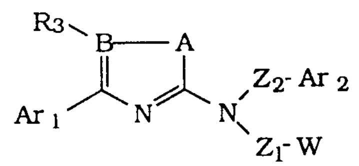

I

in der

A O oder S;

B C oder N;

$Z_1$ $C_1$-$C_4$-Alkylen oder Phenylen;

$Z_2$ $C_1$ -$C_4$-Alkylen;

W $NR_1R_2$, worin $R_1$ H, $C_1$-$C_4$-Alkyl und $R_2$ H, $C_1$-$C_4$-Alkyl, $CONQ_1Q_2$ oder $CSNQ_1Q_2$, worin $Q_1$ und $Q_2$ unabhängig voneinander H oder $C_1$-$C_4$-Alkyl darstellen, $SO_2Q_3$ oder $COQ_3$, worin $Q_3$ $C_1$-$C_4$-Alkyl darstellt, $COOQ_4$, worin $Q_4$ $C_1$-$C_4$-Alkyl oder Benzyl darstellt, oder $R_1$ und $R_2$ gemeinsam mit N einen gesättigten Morpholin-, Pyrrolidin-, Piperidin-, Piperazin- oder 4-($C_1$-$C_3$)-Alkyl-piperazin-Heterocyclus darstellen oder

W das N-Oxid der Amine $NR_1R_2$ oder W $C_1$-$C_4$-Alkoxy oder -Thioalkoxy, $CONQ_1Q_2$, $CSNQ_1Q_2$, Pyridyl, Imidazolyl oder $COOQ_5$, worin $Q_5$ $C_1$-$C_5$-Alkyl darstellt,

$R_3$ nicht vorhanden ist, wenn B N darstellt oder H, $C_1$-$C_8$-Alkyl oder Halogen, wenn B C darstellt;

$Ar_1$ Phenyl, das gegebenenfalls durch eine oder mehrere Gruppen ausgewählt aus Halogen, $C_1$-$C_4$-Alkyl, -Alkoxy oder -Thioalkoxy, Hydroxy, Carboxy, $COOQ_6$ oder $COSQ_6$ oder $CSOQ_6$, worin $Q_6$ $C_1$-$C_4$-Alkyl darstellt, Carboxamido, Cyano, Aminoacetamido, Nitro oder Trifluormethyl substituiert ist oder $Ar_1$ einen aromatischen Thienyl-, Furyl-, Indolyl-Heterocyclus oder $Ar_1$ Naphthyl, Benzyl oder Cyclohexyl oder $Ar_1$ und

$R_3$ gemeinsam eine Gruppe der Formel

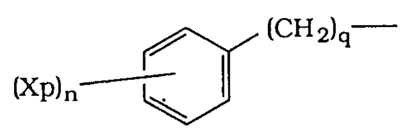

derart, daß das Kohlenstoffatom des Phenylrests in der 4-Stellung des Heterocyclus gebunden ist und worin q 2 bis 4, Xp, die gleichartig oder verschieden sind, H, $C_1$-$C_3$-Alkyl oder Halogen und n 1 bis 3 darstellen;

$Ar_2$ einen stickstoffhaltigen aromatischen Pyrimidinyl-, Chinolyl-, Isochinolyl-, Indolyl-, Isoindolyl- oder Pyridyl-Heterocyclus, der gegebenenfalls durch $C_1$-$C_3$-Alkyl oder -Alkoxy oder durch Halogen substituiert ist;

bedeuten, die Salze dieser Verbindungen mit Säuren oder Basen und wenn A, B, $R_3$, $Ar_1$, $Ar_2$, $Z_1$ und $Z_2$ die folgenden Bedeutungen besitzen:

A ist S,

B ist C,

$Z_1$ besitzt die oben angegebenen Bedeutungen,

$Z_2$ bedeutet eine $C_1$-$C_3$-Alkylengruppe,

$R_3$ bedeutet ein Wasserstoffatom oder eine $C_1$-$C_8$-Alkylgruppe,

$Ar_1$ bedeutet eine Phenylgruppe, die gegebenenfalls einen oder mehrere Substituenten ausgewählt aus Halogenatomen, $C_1$-$C_4$-Alkylgruppen oder -Alkoxygruppen, Nitrogruppen, Trifluormethylgruppen oder Hydroxylgruppen, trägt, oder $Ar_1$ und $R_3$ gemeinsam die Gruppe bilden:

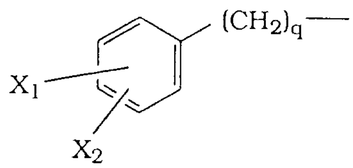

in der q den Wert 2 bis 4 besitzt und $X_1$ und $X_2$ unabhängig voneinander Wasserstoffatome, Halogenatome oder Methylgruppen darstellen, und
$Ar_2$ eine Pyrimidinyl-, Chinolyl-, Isochinolyl-, Indolyl- oder Pyridyl-gruppe, die gegebenenfalls durch eine $C_1$-$C_3$-Alkylgruppe oder -Alkoxygruppe oder ein Halogenatom substituiert ist, darstellt und
W eine Gruppe $NR_1R_2$ darstellt, worin $R_1$ und $R_2$ unabhängig voneinander Wasserstoffatome, $C_1$-$C_4$-Alkylgruppen oder Phenylgruppen oder $R_1$ und $R_2$ gemeinsam mit dem Stickstoffatom, an das sie gebunden sind, einen gesättigten Heterocyclus, wie Morpholin, Pyrrolidin, Piperidin, Piperazin oder 4-($C_1$-$C_3$)-Alkyl-piperazin darstellen oder W eine Pyridyl- oder Imidazolyl-gruppe bedeutet, sowie die Additionssalze dieser letzteren Verbindungen mit pharmazeutisch annehmbaren Säuren.

**2.** Verbindung nach Anspruch 1 der Formel I, worin B ein Kohlenstoffatom darstellt, und deren Salze.

**3.** Verbindung nach Anspruch 1 der Formel I, worin B ein Kohlenstoffatom und A Schwefel bedeuten, sowie ihre Salze.

**4.** Verbindung nach Anspruch 1 der Formel I, worin B = C, A = S und $Ar_1$ eine mindestens ortho-substituierte Phenylgruppe bedeuten, und ihre Salze.

**5.** Verbindung nach Anspruch 1 der Formel I, worin B = C, A = S, $Ar_1$ eine von ortho-Substituenten freie Phenylgruppe und $R_3$ eine $C_1$-$C_3$-Alkylgruppe bedeuten, und ihre Salze.

**6.** Verbindungen nach Anspruch 1, worin $Ar_2$ eine Pyridylgruppe darstellt.

**7.** Verbindungen nach Anspruch 1, worin $Ar_2$ eine Pyrid-2-yl- oder -3-yl-gruppe, $Z_1$ eine $C_2$-$C_4$-Alkylengruppe und W die Gruppe $NR_1R_2$ bedeuten.

**8.** Verbindung nach einem der Ansprüche 1 bis 5 der Formel I, worin W eine $C_1$-$C_2$-Alkoxygruppe oder -Thioalkoxygruppe bedeutet, und ihre Salze.

**9.** Verbindung nach einem der Ansprüche 1 bis 5 der Formel I, worin W eine Aminogruppe darstellt, und ihre Salze.

**10.** Verbindung nach einem der Ansprüche 1 bis 7 der Formel I, worin $Z_1$ eine $C_2$-$C_3$-Alkylengruppe darstellt, und ihre Salze.

**11.** Verbindung nach Anspruch 1 ausgewählt aus
- 2-N-[2-N',N'-Dimethylamino-ethyl]-N-[pyrid-3-yl-methyl]-amino-4-(2,4,6-triisopropyl-phenyl)-thiazol,
- 2-N-[2-N',N'-Dimethylamino-ethyl]-N-[pyrid-3-yl-methyl]-amino-4-(2,4,6-trimethyl-phenyl)-thiazol,
- 2-N-[2-N',N'-Dimethylamino-ethyl]-N-[pyrid-3-yl-methyl]-amino-4-(2,4-dichlor-phenyl)-5-methyl-thiazol,
- 2-N-[2-N',N'-Dimethylamino-ethyl]-N-[pyrid-3-yl-methyl]-amino-4-[2,4-dichlor-phenyl)-5-methyl-oxazol,
- 2-N-[2-N',N'-Dimethylamino-ethyl)-N-[pyrid-3-yl-methyl]-amino-4-(2,4,6-triisopropyl-phenyl)-5-chlor-thiazol und
- 2-N-[2-N',N'-Dimethylamino-ethyl]-N-[pyrid-3-yl-methyl]-amino-4-(2,4,6-triisopropyl-phenyl)-5-brom-thiazol

und deren Salze.

**12.** Verfahren zur Herstellung der Verbindungen der Formel I nach den Ansprüchen 1 bis 11, **dadurch gekennzeichnet**, daß man eine Substitutionsreaktion zwischen den primären Aminen $H_2NZ_1W$ oder $H_2N$-$Z_2Ar_2$ oder dem sekundären Amin der Formel IV

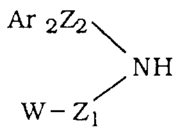

und dem Heterocyclus der Formel V

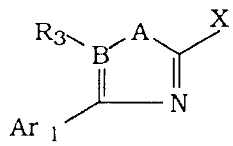

in der X ein Halogenatom oder eine Sulfonatgruppe bedeutet, durchführt, welche Reaktion dann, wenn das Amin primär ist, gefolgt wird von der Substitution des erhaltenen sekundären Amins durch Einwirkung von je nachdem W-$Z_1$-Y oder $Ar_2$-$Z_2$-Y, worin Y ein Halogenatom oder eine Sulfonatgruppe darstellt und worin A, B, $R_3$, $Ar_1$, $Z_2$, $Ar_2$, $Z_1$ und W die bezüglich der Formel I angegebenen Bedeutungen besitzen mit der Ausnahme von $R_3$ = Halogen, oder $Ar_1$, $Ar_2$ und W den entsprechenden Gruppen der Formel I entsprechen, worin die reaktiven Funktionen geschützt sind, in welchem Fall die Schutzgruppen nach der Substitution entfernt werden.

**13.** Verfahren zur Herstellung der Verbindungen der Formel I nach den Ansprüchen 1 bis 11, worin A = S und B = C bedeuten, **dadurch gekennzeichnet**, daß man einen Thioharnstoff der Formel VI

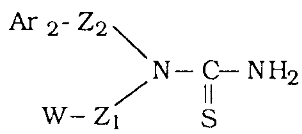

mit einem α-Halogenketon der Formel VII

$Ar_1$ - CO - CHX - $R_3$

worin $Ar_1$, $Ar_2$, $Z_1$, $Z_2$, $R_3$ und W die bezüglich der Formel I angegebenen Bedeutungen besitzen mit Ausnahme von $R_3$ = Halogen, oder entsprechende Gruppen bedeuten, worin die reaktiven Funktionen geschützt sind, in welchem Fall die Schutzgruppen nach der Kondensation entfernt werden.

**14.** Verfahren zur Herstellung der Verbindungen der Formel I der Ansprüche 1 bis 11, worin A = S und B = C bedeuten, **dadurch gekennzeichnet**, daß man ein Amin der Formel IV

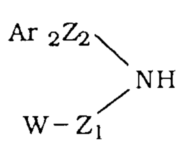

mit einem α-Thiocyanatoketon der Formel VII

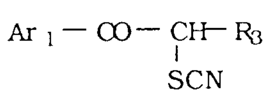

kondensiert, worin $Ar_1$, $Ar_2$, $Z_1$, $Z_2$, $R_3$ und W die bezüglich der Formel I angegebenen Bedeutungen besitzen mit der Ausnahme von $R_3$ = Halogen, oder entsprechende Gruppen bedeuten, worin die

reaktiven Gruppen geschützt worden sind, in welchem Falld ie Schutzgruppen nach der Kondensation entfernt werden.

**15.** Verfahren zur Herstellung der Verbindungen der Forme I der Ansprüche 1 bis 11, worin W $NR_1R_2$ darstellt, worin $R_2$ $CONQ_1Q_2$, $CSNQ_1Q_2$, $SO_2Q_3$ oder $COQ_3$ darstellt, worin $Q_1$, $Q_2$ und $Q_3$ die in Anspruch 1 angegebenen Bedeutungen besitzen, **dadurch gekennzeichnet**, daß man auf die entsprechende Verbindung, worin $R_2$ = H bedeutet, die man nach einem Verfahren gemäß einem der Ansprüche 12 bis 14 erhalten hat, ein reaktives Derivat von $R_2$ einwirken läßt.

**16.** Verfahren zur Herstellung der Verbindungen der Formel I der Ansprüche 1 bis 11, worin A = S oder O, B = C und $R_3$ ein Halogenatom bedeuten, **dadurch gekennzeichnet**, daß man die Halogenierung der entsprechenden Verbindung der Formel I, in der $R_3$ = H bedeutet, die man nach einem Verfahren gemäß einem der Ansprüche 12 bis 15 erhalten hat, mit dem Halogen bewirkt.

**17.** Pharmazeutische Zubereitung, **dadurch gekennzeichnet**, daß sie mindestens eine Verbindung nach einem der Ansprüche 1 bis 11, worin die Salze pharmazeutisch annehmbar sind, und mindestens ein Trägermaterial umfaßt.

**18.** Pharmazeutische Zubereitung nach Anspruch 17 zur Behandlung von pathologischen Zuständen, die mit einem PAF-Acether-Überschuß verknüpft sind, enthaltend eine wirksame Menge einer Verbindung nach einem der Ansprüche 1 bis 11.